# EUROPEAN PATENT APPLICATION

(11) **EP 1 447 446 A1**
(43) Date of publication of application: **18.08.2004**
(21) Application number: 02788584.7
(22) Date of filing: 22.10.2002
(51) Int. Cl.: C12N 15/09, C12Q 1/68

(54) **METHOD OF LABELING NUCLEIC ACIDS**

(30) Priority: 22.10.2001 JP 2001324253
(71) Applicant: TAKARA BIO INC., Otsu-shi, Shiga 520-2193 (JP)
(72) Inventor: YOSHIZAKI, Miwa, Kusatsu-shi, Shiga 525-0031 (JP); YOSHIKAWA, Yoshie, Kyoto-shi, Kyoto 607-8162 (JP); MINENO, Junichi, Uji-shi, Kyoto 611-0002 (JP); MUKAI, Hiroyuki, Moriyama-shi, Shiga 524-0102 (JP); ASADA, Kiyozo, Koka-gun, Shiga 520-3333 (JP); KATO, Ikunoshin, Koka-gun, Shiga 529-1851 (JP)
(74) Representative: VOSSIUS & PARTNER
(86) International application number: PCT/JP2002/010907
(87) International publication number: WO 2003/035865

(57) **Abstract**

The present invention relates to a method for labeling a nucleic acid characterized in that a ratio of signal intensities of each of labels of the labeled nucleic acids prepared from the same nucleic acid used as a template is substantially the same, irrelevant to the kinds of nucleic acids used as the template, a labeled nucleic acid prepared by the method, and a kit used for the method.

## Description

### TECHNICAL FIELD

The present invention relates to a method for preparing a labeled nucleic acid used in hybridization and a kit for the method, capable of performing gene expression analysis simply, quickly and at high reliability using a DNA chip or DNA microarray.

### BACKGROUND ART

While all cells of an organism have a set of genes that are inherent in the organism, the kinds and amounts of genes expressed vary depending on the kinds of cells and cellular cycle. The patterns of the kinds and amounts of genes expressed in each cell or tissue are referred to as gene expression profile. There has been considered that the functions and characteristics of each cell are determined depending on the kinds and distributions of proteins existing in the cell at the time. There has been considered, therefore, that the functions and characteristics of the cell can be deduced from the analysis of the gene expression profile capable of measuring the amounts of synthesized proteins.

Also, it has been known that the gene expression profile may significantly change from that of a normal cell due to somatic change such as a disease. In other words, causal genes or genes that are used as diagnostic indices can be found from the analysis of the gene expression profile depending upon the disease or the like.

In recent years, there has been developed a DNA chip, a DNA microarray or the like capable of measuring simultaneously the expression of a large number of genes, wherein a large number of DNAs corresponding to a large number of genes are immobilized on a substrate made of glass or the like. Therefore, the gene expression profile can be measured.

The gene expression analysis using the above DNA chip or DNA microarray is usually performed by hybridization of a probe obtained by labeling cDNA with fluorescence, wherein the cDNA is derived from mRNA prepared from a cell to be analyzed, and a DNA chip or DNA microarray. In this analysis, there have been known (1) a single color method, wherein analysis is carried out using one kind of a fluorescent substance for labeling and two DNA chips or DNA microarrays which differ for each of the two kinds of samples to be compared, and (2) a dual color hybridization method (dual color method), wherein one DNA chip or DNA microarray is competitively hybridized with a probe obtained by labeling each cDNA with two kinds of fluorescent substances having different detection wavelengths, wherein the cDNA is derived from mRNA prepared from two kinds of cells to be compared.

The analysis according to the above single color method has a drawback that it is difficult to obtain an accurate gene expression ratio because there are some possibilities that the amount and state of DNA immobilized slightly vary for each of the DNA microarrays to be used, depending upon their preparation methods. In addition, there is a drawback that accurate comparison is difficult because the background intensities vary for each of the DNA chips and the DNA microarrays. Therefore, the analysis is generally carried out according to the dual color method.

The method for labeling a cDNA probe used in the above dual color method includes a direct labeling method [P. Hedge et al., *BioTechniques*, **29(3)**, 548-562 (2000)] and an indirect labeling method [D. D. Shoemaker et al., *Nature,* **409**, 922-927 (2001)]. The direct labeling method, which is a method comprising reverse-transcribing mRNA, and incorporating a fluorescent substrate into cDNA during the cDNA synthesis, is simple. By contrast, the indirect labeling method is a labeling method comprising firstly synthesizing and purifying a non-labeled cDNA, and thereafter labeling the cDNA with a fluorescent dye by chemical reaction. An example of the above indirect labeling method includes a method comprising preparing a first-strand cDNA using a substrate having amino group during the reverse transcription of mRNA, and binding a fluorescent dye to the amino group. However, the indirect labeling method has some drawbacks in that procedures are complicated, that the time required for the preparation of the probe is long, and that purification procedures for cDNA need much steps, so that a final yield for the probe becomes low.

Therefore, there is currently most generally used a dual color method using a directly labeled cDNA probe [P. Hedge et al., *BioTechniques,* **29(3)**, 548-562 (2000)]. In the dual color method using the directly labeled cDNA probe, fluorescent substrates used for labeling are generally Cy3-labeled dUTP (or Cy3-labeled dCTP) and Cy5-labeled dUTP (or Cy5-labeled dCTP). The above direct labeling is carried out by reverse-transcribing mRNA with a reverse transcriptase in the copresence of four kinds of non-labeled substrates (dATP, dGTP, dCTP and dTTP) and Cy3-labeled dUTP (or Cy3-labeled dCTP) or Cy5-labeled dUTP (or Cy5-labeled dCTP), thereby synthesizing a first-strand cDNA.

There has been known that the length and the amount of the resulting first-strand cDNA in the above labeling vary depending on the fluorescent substrate used. For this reason, even if the mRNA of the same amount and the same molecular species exists in each sample, the fluorescent labeling ratio of the cDNA probe synthesized thereby (a ratio of the labeled signal intensity of Cy3-labeled nucleic acid to the labeled signal intensity of Cy5-labeled nucleic acid) differs for each gene. Therefore, the signal intensity ratio of the cDNA probes bound to a complementary fragment on the DNA chip or DNA microarray differs for each gene, thereby showing different apparent expression ratios. For instance, even if the same mRNA exists in each of two or more kinds of samples to be determined, when each mRNA is labeled with a different kind of a fluorescent substance, the ratio of the amounts of these fluorescent substrates incorporated into the labeled cDNA probe may differ for each gene. Therefore, the conventional dual color method using the directly labeled cDNA probe as mentioned above has a drawback that the ratio of the signal intensity ascribed to the labeling of the cDNA probe hybridized to a DNA on a DNA chip or DNA microarray differs for each gene, whereby showing different apparent expression ratios. In other words, if the dual color hybridization is carried out using a cDNA probe labeled with different labeled substrates using the same mRNA in the same amount, followed by correction analysis (for instance, global normalization), the expression of a case where the labeling is carried out with one labeling substrate and a case where the labeling is carried out with another labeling substrate is supposed to be substantially the same for all of the genes, but there are some cases where the genes not having substantially the same expression are generated.

Moreover, in order to carry out a method for preparing a directly labeled cDNA probe, there has been commercially available a kit comprising a reverse transcriptase so that a correction is made on the difference in incorporation ratio between different labeled substrates in the same nucleic acid used as a template. However, even if the above kit is used, there is a drawback that the ratio of the incorporation efficiencies of different labeled substrates, for instance, a ratio of the incorporation efficiency of a Cy3-labeled substrate to the incorporation efficiency of a Cy5-labeled substrate, cannot be made at the same level for all of the genes. Furthermore, according to the above kit, there are many cases where the ratio of the signal intensity of a signal ascribed to a Cy3-labeled nucleic acid to a signal ascribed to a Cy5-labeled nucleic acid, each labeled nucleic acid being prepared from the same nucleic acid used as a template, may fluctuate depending upon the kinds of nucleic acids used as the template. Therefore, the ratio of the labeled signal intensity after the subsequent global normalization treatment may not reflect the original abundance ratio of the nucleic acid as a template in some cases.

In the gene expression analysis using a DNA chip or DNA microarray, there are many cases where the expression alterations of 2 folds or more or 1/2 folds or less that of the control sample (control mRNA) is considered as a significant expression alteration. However, even if the same mRNA is used in the same amount, in the case of a labeling method involving a gene of which expression level is calculated to be 2 folds or more or 1/2 folds or less that of another gene, totally erroneous results are obtained so that it is difficult to obtain an accurate alteration of gene expression.

In addition, when a labeled substrate is incorporated into a nucleic acid in a general enzyme reaction, improvement of an incorporation efficiency has been tried by lowering a concentration of the non-labeled substrate and increasing a concentration of the labeled substrate, within a range in which the reaction ability of the enzyme can be maintained at a given level. In the labeling of a cDNA probe used in the dual color hybridization method, the concentrations of the non-labeled substrate and the labeled substrate have been also taken into consideration in order to increase the incorporation of the labeled substrate into the nucleic acid as described above. However, the above-mentioned drawbacks inherently owned by the dual color hybridization method have not been taken into consideration in the current situation.

Therefore, there has been desired a method for labeling a target nucleic acid capable of understanding the behavior of an accurate gene expression in the gene expression analysis.

### DISCLOSURE OF INVENTION

An object of the present invention is to provide a method for labeling a nucleic acid characterized in that a ratio of signal intensities of each of labels of the labeled nucleic acids prepared from the same nucleic acid used as a template is substantially the same, irrelevant to the kinds of nucleic acids used as the template, a labeled nucleic acid prepared by the method, and a kit for the method.

A first invention of the present invention relates to a method for labeling each of nucleic acids in a nucleic acid sample containing plural nucleic acids with at least two kinds of different labeled substances distinguishable from each other, characterized in that a ratio of signal intensities of each of labels of the labeled nucleic acids is substantially the same, irrelevant to the kinds of nucleic acids used as the template, when the labeled nucleic acids are prepared from arbitrary nucleic acids as a template, and the labeled nucleic acids are labeled with the labeled substrate. More concretely, the first invention relates to a method for labeling a nucleic acid, wherein the method is a method for labeling the nucleic acid with at least two kinds of different labeled substances distinguishable from each other, and wherein the method comprises the step of labeling the nucleic acid in a nucleic acid sample containing plural kinds of nucleic acids by use of:
one labeled substrate which is labeled with a labeling substance and
a non-labeled substrate corresponding thereto,
in an amount ratio satisfying the following conditions that a ratio of:
a) a signal intensity of a signal ascribed to a labeled nucleic acid prepared with the nucleic acid in the nucleic acid samples as a template, wherein the labeled nucleic acid is labeled with the labeled substrate, to
b) a signal intensity of a signal ascribed to a labeled nucleic acid prepared with the same nucleic acid as that of the above item a) as a template, wherein the labeled nucleic acid is labeled with a labeled substrate different from the labeled substrate of the above item a)
in each of the nucleic acids in the nucleic acid sample is substantially the same, irrelevant to the kinds of the nucleic acids to be used as a template.

In the first invention of the present invention, it is desired that a labeled nucleic acid may be preferably prepared by reverse transcription reaction from the nucleic acid used as a template, and that the different labeled substrate is preferably a Cy3-labeled substrate or a Cy5-labeled substrate. There can be preferably used a reaction mixture used for labeling comprising the non-labeled substrate and the Cy3-labeled substrate, wherein its ratio within the range of from 1:1 to 5:1, and/or a reaction mixture used for labeling comprising the non-labeled substrate and the Cy5-labeled substrate, wherein its ratio within the range of from 3:1 to 10:1. In other words, in the first invention of the present invention, it is desired that the nucleic acids in the nucleic acid sample are labeled in a reaction mixture containing the non-labeled substrate and the Cy3-labeled substrate preferably in a concentration ratio (the non-labeled substrate/the Cy3-labeled substrate) within the range of from 1/1 to 5/1. In addition, it is desired that the nucleic acids in the nucleic acid sample are labeled in a reaction mixture containing the non-labeled substrate and the Cy5-labeled substrate in a concentration ratio (the non-labeled substrate/the Cy5-labeled substrate) within the range of from 3/1 to 10/1.

A second invention of the present invention relates to a labeled nucleic acid prepared by the method for labeling a nucleic acid of the first invention of the present invention.

A third invention of the present invention relates to a kit for labeling a nucleic acid comprising an instruction manual describing the method for labeling a nucleic acid of the first invention of the present invention, i.e. a kit comprising an instruction manual describing the procedures of the method for labeling a nucleic acid of the first invention of the present invention.

In the third invention of the present invention, there can be preferably used a kit comprising an instruction manual describing a method for preparing a mixed substrate by use of the non-labeled substrate and the Cy3-labeled substrate, wherein the concentration ratio thereof (the non-labeled substrate/the Cy3-labeled substrate) ranges from 1/1 to 5/1; and/or a kit comprising an instruction manual describing a method for preparing a mixed substrate by use of the non-labeled substrate and the Cy5-labeled substrate, wherein the concentration ratio thereof (the non-labeled substrate/the Cy5-labeled substrate) ranges from 3/1 to 10/1.

Furthermore, a fourth invention of the present invention relates to a kit for labeling a nucleic acid, comprising:
(1) a reaction vessel containing a reaction mixture comprising a Cy3-labeled substrate and a non-labeled substrate corresponding thereto in a concentration ratio (non-labeled substrate/Cy3-labeled substrate) of from 1/1 to 5/1, and comprises a non-labeled nucleotide substrate other than the above non-labeled substrate, and/or
(2) a reaction vessel containing a reaction mixture comprising a Cy5-labeled substrate and a non-labeled substrate corresponding thereto in a concentration ratio (non-labeled substrate/Cy5-labeled substrate) within the range of from 3/1 to 10/1, and comprises a non-labeled nucleotide substrate other than the above non-labeled substrate,
wherein the reaction mixture is a reaction mixture for one-time use or defined times of use. The above kit for labeling a nucleic acid may further comprise a reverse transcriptase.

### BRIEF DESCRIPTION OF THE DRAWINGS

Figure 1 is a diagram showing "Scatter Plot" of the fluorescent intensity in a case where a fluorescent-labeled probe prepared by the method of the present invention is used.
Figure 2 is a diagram showing "Scatter Plot" of the fluorescent intensity in a case where a fluorescent-labeled probe prepared by a conventional method is used.
Figure 3 is a diagram showing "Scatter Plot" of the fluorescent intensity in a case where a fluorescent-labeled probe prepared with a commercially available kit is used.

### BEST MODE FOR CARRYING OUT THE INVENTION

The present inventors have found that labeling of a target nucleic acid, capable of understanding the behavior of gene expression surprisingly accurately in gene expression analysis, can be achieved by setting a concentration ratio of a non-labeled substrate to a labeled substrate at a particular ratio for each of at least two kinds of labeled substrates, for instance, fluorescent substrates. They have further found that according to the labeling method based on the concentration ratio, a kit for fluorescent-labeling a probe capable of performing accurate gene expression analysis using the dual color hybridization method in high accuracy, whereby the present inventors have achieved the present invention.

In the present specification, the evaluation of the method for labeling a nucleic acid is carried out by, for instance, the steps of:
(1) carrying out a reverse transcription reaction using at least two kinds of different labeled substrates with the same amount of mRNA as a template for each of the labeled substrates to give a labeled cDNA,
(2) carrying out hybridization using each of the labeled cDNAs obtained in the above step (1) and a DNA chip or DNA microarray, and
(3) calculating an extent of variance of the ratios of signal intensities between the signals ascribed to each of the labeled substrates for spots showing significant signal intensity for all of the labeled substrates by correction analysis of a signal ascribed to the labeled substrate of each complex, wherein the complex is formed by the hybridization in the above step (2) (a complex of the labeled cDNA with a DNA which is immobilized on the DNA chip or DNA microarray).

Concretely, for instance, the method for labeling a nucleic acid of the present invention can be evaluated by using as an index an extent of variance of a ratio of signal intensities of the spots showing significant signal intensity for both Cy3 and Cy5, when the same mRNA in the same amount is used for labeling with different labeled substrates, for instance, fluorescent substrates, concretely CyDye-labeled substrates (Cy3-labeled substrate and Cy5-labeled substrate), dual color hybridization is carried out, and thereafter a signal intensity ascribed to Cy5 is subjected to correction analysis against to a signal intensity ascribed to Cy3.

In the present specification, the above extent of variance refers to a value calculated by [standard deviation of (logarithmic value of Cy3/Cy5 ratio) x 2.5].

In addition, in the above evaluation, when the above value showing the extent of variance is 2 or less, it is shown that the labeling method is a labeling method capable of understanding accurately the behavior of gene expression.

In the present specification, "substantially the same" refers to the fact that in the scatter plot of signal intensity of label ascribed to each of the labeled nucleic acids labeled with labeling substances distinguishable from each other, the signal intensity of the signal ascribed to arbitrary one labeling substance / the signal intensity of the signal ascribed to another labeling substance falls within the range of 1/2 to 2/1.

In the present specification, the "signal intensity" refers to a value obtained by subjecting a DNA chip or DNA microarray to a fluorescence reader (array scanner) to measure the spots showing fluorescence, wherein DNA chip or DNA microarray has been hybridized with labeled nucleic acids (probes), thereafter washed and dried, and subtracting an average intensity of the background signals in the surroundings of each spot from an average intensity of the fluorescence signals showing the spots which are immobilized regions of the nucleic acids on a DNA chip or DNA microarray, when the fluorescence signal intensity of each spot is determined with a quantitative image analytical software program.

In the present specification, the "significant signal intensity" refers to a case where [Mean of a given one spot signal] shows a value greater than [Mean + 2 x SD (standard deviation) of the background signal] of the surroundings of each spot.

In the present specification, the correction of a signal intensity of a signal ascribed to a labeling substance, for instance, a signal intensity of a signal ascribed to a fluorescent substance, concretely a signal intensity of a signal ascribed to Cy3 and a signal intensity of a signal ascribed to Cy5, can be carried out by using global normalization method. Concretely, for instance, the signal intensity ascribed to Cy5 is corrected so that logarithmic values of the ratio of the signal intensity of the signal ascribed Cy3 / the signal intensity of the signal ascribed to Cy5 has an average value of 0, wherein the values are taken for each of the genes having a significant signal intensity in the sample to be measured.

In the present specification, the "extent of variance" uses as an index a value calculated by a constant obtained by [standard deviation of (logarithmic value of Cy3/Cy5 ratio) x 2.5], when a histogram of the logarithmic value of the Cy3/Cy5 ratio for the spots showing significant signal intensity for labeling substances, for instance, fluorescent substances, concretely for both Cy3 and Cy5, is drawn, wherein the histogram shows a nearly normal distribution.

The method for calculating the index for the above "extent of variance" utilizes the fact that 99% of numerical values fall within 2.5 standard deviations from the mean, when it is supposed that the data show a normal distribution. The index represents the variance from the mean in which the great majority of spots are distributed excluding few exceptional spots.

In the present specification, the "labeled substrate" refers to a substance in which a labeling substance, for instance, a fluorescent substance, a radioactive compound, biotin, amino group or the like is added to a nucleotide substrate, and includes concretely Cy5-dUTP, Cy5-dCTP, Cy3-dUTP, and Cy3-dCTP.

The above "nucleotide substrate" includes a substrate usable in nucleic acid synthesis, concretely dATP, dGTP, dCTP, dTTP, dUTP or the like. In the present specification, the representation "nucleotide substrate" means a non-labeled substrate unless specified otherwise.

In the present specification, the phrase "non-labeled substrate corresponding to a labeled substrate" or the term "non-labeled substrate" refers to a nucleotide substrate which is incorporated in place of the above labeled substrate. There are included, for instance, but not particularly limited to, dTTP when a labeled substrate is a labeled dUTP or the like, concretely, for instance, Cy3-dUTP or Cy5-dUTP; and dCTP when a labeled substrate is a labeled dCTP or the like, concretely, for instance, Cy3-dCTP or Cy5-dCTP.

In the present specification, "plural" in the phrase "nucleic acid sample containing plural kinds of nucleic acids" has the same definition as two kinds or more.

The present invention will be hereinafter described in detail.

### (1) Method for Labeling Nucleic Acid of the Present Invention and Labeled

### Nucleic Acids Prepared by the Method

The method for labeling a nucleic acid of the present invention is a method for labeling each of nucleic acids in a nucleic acid sample containing plural kinds of nucleic acids with two or more different labeled substances distinguishable from each other, wherein one of the significant features of the method resides in that a ratio of each of labeled signal intensities of the labeled nucleic acids is substantially the same, irrelevant to the kinds of nucleic acids used as the template, wherein the labeled nucleic acids are those prepared from arbitrary nucleic acids as a template, and labeled with the labeled substrate. In other words, the labeling method of the present invention is a method for labeling a nucleic acid with at least two kinds of different labeling substances distinguishable from each other, wherein one of the features of the method resides in that there is carried out the step of labeling the nucleic acid (concretely all nucleic acids or a part of nucleic acids) in a nucleic acid sample containing plural kinds of nucleic acids are labeled by use of:
one labeled substrate which is labeled with a labeling substance and
a non-labeled substrate corresponding thereto,
in an amount ratio satisfying the following conditions that a ratio of:
a) a signal intensity of a signal ascribed to a labeled nucleic acid prepared with the nucleic acid in the nucleic acid samples as a template, wherein the labeled nucleic acid is labeled with the labeled substrate, to
b) a signal intensity of a signal ascribed to a labeled nucleic acid prepared with the same nucleic acid as that of the above a) as a template, wherein the labeled nucleic acid is labeled with a labeled substrate different from the labeled substrate of the above a)
in each of the nucleic acids in the nucleic acid sample is substantially the same, irrelevant to the kinds of the nucleic acids to be used as a template.

According to the labeling method of the present invention, since the non-labeled substrate and the labeled substrate are used at the above content ratio, in a case where the same mRNA is used as a template, there is exhibited an excellent effect that substantially the same expression ratio (Cy3 signal/Cy5 signal ratio) can be obtained, irrelevant to the kinds of genes. In addition, according to the labeling method of the present invention, since the non-labeled substrate and the labeled substrate are used at the above content ratio, there is exhibited an excellent effect that the behavior of gene expression can be understood accurately.

According to the method for labeling a nucleic acid of the present invention, the nucleic acid to be used as a template can be prepared from a sample possibly containing a nucleic acid such as DNA or RNA. The above samples include, for instance, but are not particularly limited to, biological samples such as whole blood, serum, buffy coat, urine, feces, cerebrospinal fluid, semen, saliva, tissues (for instance, cancer tissue, lymph nodes and the like), and cell cultures (for instance, mammalian cell cultures, bacterial cultures and the like); nucleic acid-containing samples such as of viroids, viruses, bacteria, fungi, yeast, plants, and animals; samples possibly contaminated or infected with microorganisms such as viruses or bacteria (foods, biological products, and the like); or samples possibly containing organisms, such as soil and wastewater. The samples include samples obtained by appropriately treating each of the biological samples, the nucleic acid-containing samples, the samples potentially contaminated or infected with microorganisms and the samples possibly containing organisms mentioned above.

As the above "nucleic acid as a template," any of RNAs and DNAs can be preferably used. When the analysis of gene expression is carried out in a cell, mRNA obtained from the cell can be used as the above "nucleic acid as a template."

The method for labeling a nucleic acid of the present invention can be applied to any of nucleic acids, as long as the nucleic acids are nucleic acids capable of labeling with a commonly used labeling substance. The method for labeling a nucleic acid of the present invention can, for instance, be used, but not particularly limited to, during the first strand cDNA synthesis reaction (reverse transcription reaction).

The reverse transcriptase which can be used in the above reverse transcription reaction includes, for instance, but is not particularly limited to, AMV RTase, MMLV RTase, RAV-2 RTase and the like.

In addition, in the labeling method of the present invention, when a CyDye-labeled substrate, for instance, is used, it is desirable that a final concentration of the CyDye-labeled substrate in a reaction mixture is, but not particularly limited to, preferably within the range of 0.02 mM to 0.3 mM, more preferably within the range of 0.025 mM to 0.1 mM, from the viewpoint of obtaining an excellent economic advantage and high operability, concretely, for instance, from the viewpoint of easily completely removing an unreacted CyDye-labeled substrate from a solution containing a labeled nucleic acid in the purification step, thereby giving an excellent background, and from the viewpoint of incorporating a labeled substrate into a nucleic acid in an amount sufficient for the enzyme reaction, thereby improving a signal intensity of the resulting labeled nucleic acids.

The method for labeling a nucleic acid of the present invention can be carried out by optimizing a concentration ratio of the non-labeled substrate to the labeled substrate used during the fluorescence-labeled cDNA probe synthesis reaction using mRNA (i.e., reverse transcription reaction). The ratio for the above non-labeled substrate/labeled substrate is expressed as, for instance, a concentration ratio of CyDye-labeled dUTP/dTTP in the use of CyDye-labeled dUTP, or as a concentration ratio of CyDye-labeled dCTP/dCTP in the use of CyDye-labeled dCTP.

In the labeling method of the present invention, it is also desirable that the amounts of the nucleotide substrates used for nucleic acid synthesis, concretely dATP, dGTP, dCTP, and dTTP (including dUTP) are preferably of equivalence. Concretely, it is desirable that the total amount thereof (concentration) are of equivalence between a case of the labeled substrates used and corresponding non-labeled substrates thereof, and a case of other nucleotide substrates. In a embodiment of the present invention, in a case where, for instance, the CyDye-labeled dUTP is used at a concentration of 0.05 mM in a reaction mixture, it is preferable, but not particularly limited to, that dTTP is used at a concentration of 0.1 mM in a reaction mixture (a combined concentration of the labeled dUTP and dTTP being 0.15 mM), and that each of dATP, dGTP and dCTP is used at a concentration of 0.15 mM in the reaction mixture when the ratio of the non-labeled substrate/labeled substrate is 2.

As described above, the labeling method of the present invention can be evaluated by determining whether or not there is a difference in a variance of signal intensity ratio of each spot, or a ratio of spots each having a significant signal, due to the difference in the kind of a fluorescent dye Cy3 or Cy5 used for labeling, when the labeled probe is prepared by varying a ratio of the non-labeled substrate/labeled substrate with fixing a concentration of the CyDye-labeled substrate in the above concentration range.

The above "ratio of spots having a significant signal" is used as an index for the signal intensity. Here, the larger the number of the significant spots is, the larger the ratio of spots having a significant signal intensity against the surrounding background, and the data for expression alterations (expression profile) are obtained with greater significance for a larger number of genes, whereby making it preferable for the analysis.

Furthermore, the labeling method of the present invention can be evaluated for each of the Cy3-labeled substrate and the Cy5-labeled substrate from the viewpoint of evenness in the signal intensity ratio for each of the genes, by preparing labeled nucleic acids (probes) with varying a ratio of the non-labeled substrate/labeled substrate against a given concentration of the Cy3-labeled substrate and Cy5-labeled substrate, and carrying out dual color hybridization analysis using all ratios for each substrate.

In the labeling method of the present invention, it is desirable, for instance, but not particularly limited to, that in a case where the same mRNA is used as a template, the range for the concentration ratio of the non-labeled substrate/labeled substrate is preferably 1/1 to 5/1, especially preferably 1.5/1 to 4/1, in a Cy3-labeled substrate, and that the range is preferably 3/1 to 10/1, especially preferably 4.5/1 to 9/1, for a Cy5-labeled substrate, from the viewpoint of obtaining substantially the same expression ratio (ratio of Cy3 signal/Cy5 signal), irrelevant to the kinds of the genes.

According to the labeling method of the present invention, there is exhibited an excellent effect that there can be prepared a labeled nucleic acid having an abundance ratio inherently owned by the nucleic acid used as a template in a nucleic acid-containing sample, for instance, but not particularly limited to, an abundance ratio of mRNA. Therefore, labeled nucleic acids prepared by the labeling method of the present invention are also encompassed in the present invention.

The labeled nucleic acid of the present invention can be utilized for all of the methods, as long as the hybridization is carried out according to the dual color method. For instance, since a nucleic acid labeled by the labeling method of the present invention has an inherent abundance ratio between two kinds of samples in the nucleic acid-containing sample, there can be preferably used in, for instance, but not particularly limited to, a hybridization method using a DNA microarray.

In addition, according to the labeling method of the present invention, the accuracy of the analysis of expressed genes can be improved in gene expression analysis according to the dual color hybridization method because expression alterations of 1.5 folds can be judged to be a significant alternation at a probability of about 99%, in contrast to alterations of 2 folds or more have been judged to be a significant difference in expression in the conventional method.

The labeled nucleic acid of the present invention is a nucleic acid used as a template in a nucleic acid sample containing plural kinds of nucleic acids, for instance, but not particularly limited to, a labeled nucleic acid having an abundance ratio of mRNA. Further, a feature of the labeled nucleic acid of the present invention resides in that the signal intensity ratio of each signal ascribed to labeled nucleic acids prepared from a given nucleic acid as a template is substantially the same, wherein the labeled nucleic acids have different labeled substrates, irrelevant to the kinds of the nucleic acids used as a template.

Furthermore, by using the labeling method of the present invention, the number of genes which can be analyzed at high reliability can be increased under the conditions that give as large a ratio of the significant spots as possible. In the present invention, the accuracy of gene expression analysis using the DNA chip or DNA microarray is increased so that expression alterations of within 2 folds can be also judged as a significant difference.

Therefore, according to the labeling method of the present invention, there is provided a method for analyzing gene profile characterized by the use of the labeled nucleic acid obtained by the labeling method of the present invention.

### (2) Kit for Labeling Nucleic Acid of the Present Invention

The present invention provides a kit for labeling a nucleic acid used in the method for labeling a nucleic acid of the present invention described above.

In one embodiment of the present invention, the kit for labeling a nucleic acid of the present invention includes a kit comprising an instruction manual describing procedures for the labeling method of the present invention in a packaged form. In a preferred embodiment, the kit for labeling a nucleic acid of the present invention resides has a feature that the kit comprises an instruction manual describing a method for preparing a mixed substrate containing the Cy3-labeled substrate and the non-labeled substrate corresponding thereto in a concentration ratio (the non-labeled substrate/the Cy3-labeled substrate) within the range of from 1/1 to 5/1, preferably 1.5/1 to 4/1, and/or an instruction manual describing a method for preparing a mixed substrate containing the Cy5-labeled substrate and the non-labeled substrate corresponding thereto in a concentration ratio (the non-labeled substrate/the Cy5-labeled substrate) within the range of from 3/1 to 10/1, preferably 4.5/1 to 9/1. In addition, the kit of the present invention may comprise a non-labeled substrate and/or a labeled substrate. Furthermore, the kit of the present invention may comprise various necessary reagents including a reverse transcriptase, a reverse transcription reaction buffer and the like. Alternatively, a commercially available enzyme having reverse transcription activity may be selected and used in accordance with the instruction manual. The reverse transcriptase is not particularly limited, and AMV RTase, MMLV RTase, or RAV-2 RTase can be preferably used.

The above "instruction manual" refers to a printed matter describing a method for using the kit, for instance, a method for preparing a reverse transcription reaction reagent solution, the mixing ratio of the non-labeled substrate to the labeled substrate and their amounts, recommended reaction conditions or the like. The instruction manual includes, in addition to instruction manuals in the form of a pamphlet or leaflet, labels attached to a kit and description given on the package housing the kit. Furthermore, there is included information disclosed or provided via electronic media such as internet.

Furthermore, the kit used for a method for detecting a target nucleic acid may be a kit comprising, in addition to the above instruction manual and the reverse transcription reaction reagent, an oligonucleotide primer for the reverse transcription reaction (random primer or oligo-dT primer) or the like. The kit may further comprise a membrane filter unit for purifying the resulting labeled nucleic acid.

According to the kit for labeling a nucleic acid of the present invention, a labeled probe capable of carrying out analysis at high accuracy analysis can be prepared simply and under conditions in which the amount of CyDye-labeled substrate used is reduced.

Further, another embodiment of the kit for labeling a nucleic acid of the present invention includes a kit comprising:
(1) a reaction vessel containing a reaction mixture in an amount of one-time use or defined times of use, wherein the reaction mixture comprises a Cy3-labeled substrate and a non-labeled substrate corresponding thereto in a concentration ratio (non-labeled substrate/Cy3-labeled substrate) of from 1/1 to 5/1, preferably from 1.5/1 to 4/1, and comprises a non-labeled nucleotide substrate other than the above
   non-labeled substrate, and/or
(2) a reaction vessel containing a reaction mixture in an amount for one-time use or defined times of use, wherein the reaction mixture comprises a Cy5-labeled substrate and a non-labeled substrate corresponding thereto in a concentration ratio (non-labeled substrate/Cy5-labeled substrate) within the range of from 3/1 to 10/1, preferably from 4.5/1 to 9/1, and comprises a non-labeled nucleotide substrate other than the above non-labeled substrate.
The kit may further comprise the above instruction manual, a reverse transcriptase, reagents for reverse transcription reaction, an oligonucleotide primer for the reverse transcription reaction (random primer or oligo-dT primer), a gel filtration column for purifying the labeled nucleic acid obtained, and the like. Here, the above "reaction mixture in an amount for one-time use or defined times of use" refers to a reaction mixture in an amount suitable for carrying out the reaction once or previously determined number of times.

In the kit of the present invention, the reaction mixture may be dispensed to a single reaction vessel for one-time use (referred to as one-time reaction vessel), or may be dispensed to a single reaction vessel for the defined times of use (referred to as multiple reaction vessel). According to the above one-time reaction vessel, the labeling method of the present invention can be carried out conveniently by allowing a user to add the target nucleic acid to be labeled in a given amount as instructed according to the instruction manual or the like, and subjecting the reaction vessel under the instructed reaction conditions. According to the multiple reaction vessel, the labeling method of the present invention can be carried out conveniently by dispensing the reaction mixture in a given amount as instructed in the instruction manual or the like and the target nucleic acid to be labeled into separate reaction vessels, and subjecting the reaction vessels under the instructed reaction conditions.

The above reaction vessel includes, for instance, a 1.5 ml-capacity mini-tube, a 200 µl-capacity micro-tube, and the like, and the volume of the vessel is not limited to those exemplified above.

The present invention will be described in more detail hereinbelow by means of Examples, without intending to limit the present invention to the scope of the Examples.

### Example 1

Various concentration ratios were set for a Cy3- or Cy5-labeled substrate (Cy3-dUTP or Cy5-dUTP) and a non-labeled substrate (dTTP). Each of the substrate concentrations is shown in Table 1.

Using each substrate so as to have the substrate concentrations shown in Table 1 in each of the Cy3 and Cy5 systems, the same mRNA was used in the same amount of mRNA for labeling the mRNA, to give each of labeled cDNA probes (Cy3-labeled cDNA probe and Cy5-labeled cDNA probe). In the gene expression analysis using the labeled cDNA probes obtained and a DNA chip or DNA microarray, studies on optimization of the above concentration ratio were conducted so that a ratio of a Cy3 fluorescence signal intensity to a Cy5 fluorescence signal intensity is substantially the same for all the genes.

Concretely, the concentration ratios of the non-labeled substrate/labeled substrate were set at 5/1, 3.5/1 and 2/1 to determine the concentration ratio at which analytical results with high accuracy could be obtained.

**Table 1**

| Non-Labeled Substrate / Labeled Substrate = 5/1 | |
|---|---|
| dATP | 0.30 mM |
| dGTP | 0.30 mM |
| dCTP | 0.30 mM |
| dTTP | 0.25 mM |
| Cy-dUTP | 0.05 mM |

| Non-Labeled Substrate / Labeled Substrate = 3.5/1 | |
|---|---|
| dATP | 0.225 mM |
| dGTP | 0.225 mM |
| dCTP | 0.225 mM |
| dTTP | 0.175 mM |
| Cy-dUTP | 0.05 mM |

| Non-Labeled Substrate / Labeled Substrate = 2/1 | |
|---|---|
| dATP | 0.15 mM |
| dGTP | 0.15 mM |
| dCTP | 0.15 mM |
| dTTP | 0.10 mM |
| Cy-dUTP | 0.05 mM |

The preparation of the labeled cDNA probes were carried out as follows. First, polyA(+) RNA was prepared from human HL-60 cells using Triazol Reagent (manufactured by GIBCO BRL) and Oligotex-dT30<Super> (manufactured by Takara Bio Inc.) in accordance with the protocol for each of the kits. Each of a Cy3-labeled cDNA probe and a Cy5-labeled cDNA probe was prepared and purified using RNA Fluorescence Labeling Core Kit (M-MLV Version) (manufactured by Takara Bio Inc.) in accordance with the protocol for the kit, with 1 µg of the polyA(+) RNA obtained as a template. Here, each of the substrates of the above concentration was used in place of a 10 × dNTP Mixture contained in the above kit.

Next, hybridization between IntelliGene™ Human 1K Set I (manufactured by Takara Bio Inc.) and the above labeled cDNA probes was carried out in accordance with the instruction manual for IntelliGene™ (manufactured by Takara Bio Inc.). Thereafter, the DNA chip or DNA microarray after the hybridization was washed and dried. Subsequently, the DNA chip or DNA microarray was scanned using Affymetrix 428 Array Scanner (manufactured by Affymetrix) to obtain fluorescent images for each of Cy3 (excitation wavelength: 532 nm, detection wavelength: 570 nm) and Cy5 (excitation wavelength: 635 nm, detection wavelength: 670 nm).

Next, the signal intensity of each spot on the fluorescent images obtained was calculated using quantitative image analytical software program, ImaGene 4.0 (manufactured by BioDiscovery). The ratio of Cy3/Cy5 signal intensity for each spot was expressed as a logarithmic value, and the correction of the signal intensities between Cy3 and Cy5 was carried out by global normalization method, by which correction was made so that an average signal intensity ratio would be 0 for all spots.

The expression ratio (ratio of Cy3/Cy5 signal intensity) was calculated from the corrected signal intensity ratio, and the range of the expression ratio for 99% convergence in distribution of the spots that are significant signals for both Cy3 and Cy5 was determined. The above range of the expression ratio was used as an index of analytical accuracy. Here, those of which Mean (average value) of the spot signals shows a value greater than the value of Mean + 2 × SD (standard deviation) of the background signal of the surrounding of each spot are defined as significant signals.

The concentration ratio of the non-labeled substrate/labeled substrate, the range of the expression ratio for 99% convergence in distribution of the significant spots at the concentration ratio, and the number of significant spots (%) for each of Cy3 and Cy5 are shown in Table 2.

**Table 2**

| Non-Labeled Substrate/ Labeled Substrate | Expression Ratio | Effective Spot for Cy3 | Effective Spot for Cy5 |
|---|---|---|---|
| 5/1 | 1/2.12 - 2.12 | 38% | 43% |
| 3.5/1 | 1/2.40 - 2.40 | 45% | 47% |
| 2/1 | 1/2.86 - 2.86 | 53% | 47% |

As shown in Table 2, it was found that since the distribution of the expression ratio more closely converges to 1 when the concentration ratio of non-labeled substrate/labeled substrate is made higher (non-labeled substrate/labeled substrate = 5/1) as compared to that when the concentration ratio of the non-labeled substrate/labeled substrate is made lower (non-labeled substrate/labeled substrate = 2/1), the accuracy is increased.

### Example 2

### (1) Studies on Combinations of Concentration Ratios of Non-Labeled Substrate/Labeled Substrate

By setting the concentration ratio of the non-labeled substrate/labeled substrate high (5/1) or low (2/1), studies were conducted on which of the concentration ratios was suitable for each of Cy3 and Cy5. Here, the preparation of labeled cDNA probes, hybridization with a DNA microarray, washing, scanning and analysis were carried out under the same conditions as those described in Example 1 except that the concentration ratios of the non-labeled substrate/labeled substrate were changed. As the DNA microarray, there was used IntelliGene™ Human Cancer Chip Ver. 2.0 (manufactured by Takara Bio Inc.). The combinations studied and the results of the range of the expression ratio are shown in Table 3.

**Table 3**

| | | Cy5 | |
|---|---|---|---|
| | Non-Labeled Substrate/ Labeled Substrate | 5/1 | 2/1 |
| Cy3 | 5/1 | 1.96⁽ⁱ⁾ | 2.99⁽ⁱⁱⁱ⁾ |
| | 2/1 | 1.80⁽ⁱⁱ⁾ | 2.27^{(iv)} |

In Table 3, (iv) shows a comparative example by a conventional method [P. Hedge et al., *BioTechniques,* **29(3)**, 548-562 (2000)].

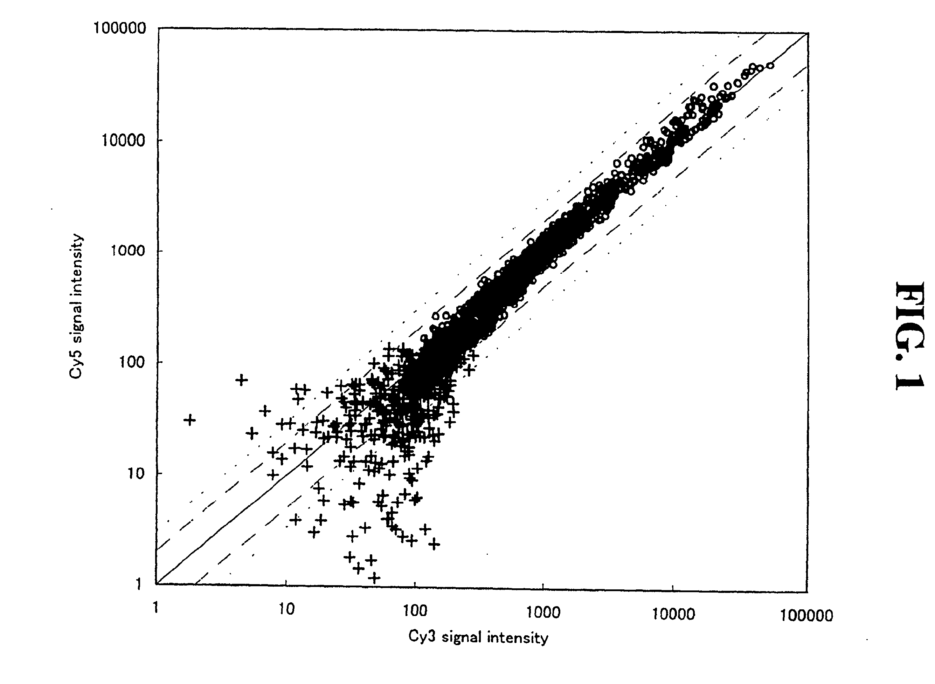

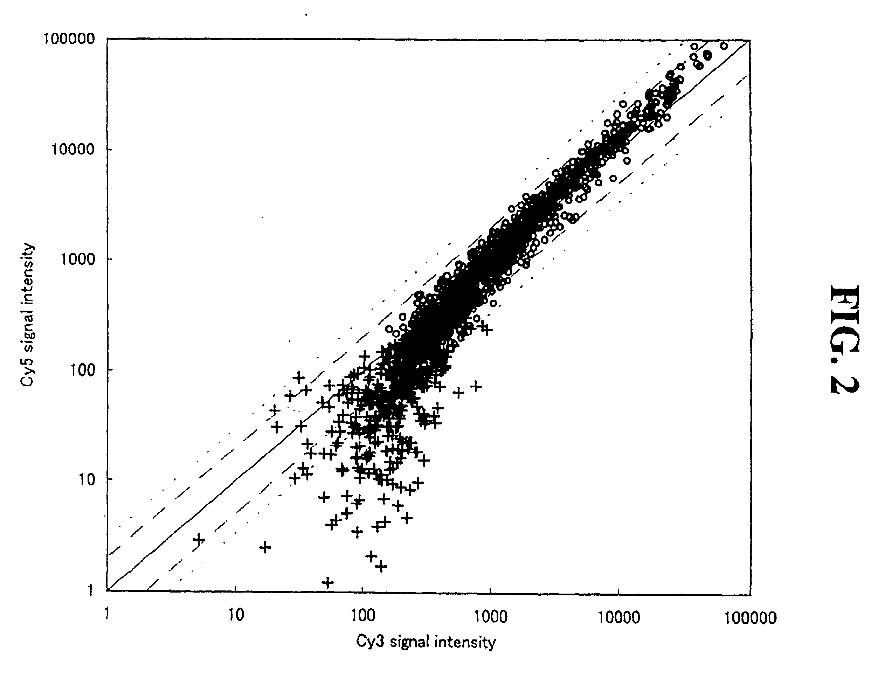

The "Scatter Plot" in a case where the concentration ratio of the non-labeled substrate/labeled substrate for Cy3 was 2/1, and where the concentration ratio of the non-labeled substrate/labeled substrate for Cy5 was 5/1 [(ii) in Table 3] is shown in Figure 1. The "Scatter Plot" in a case where the concentration ratio of the non-labeled substrate/labeled substrate for Cy3 was 2/1, and where the concentration ratio of the non-labeled substrate/labeled substrate for Cy5 was 2/1 [(iv) in Table 3] is shown in Figure 2.

Figures 1 and 2 are diagrams each showing signal intensities obtained when each fluorescent probe was used. In the figures, the X-axis is a Cy3 signal intensity, and the Y-axis is a Cy5 signal intensity, wherein an open circle (O) represents a spot exhibiting significant signal intensity; and a cross (+) represents a spot exhibiting non-significant signal intensity. Also, in the figures, a solid line is a theoretical line in which the expression ratio of the Cy3 signal intensity to the Cy5 signal intensity becomes 1:1, a broken line is a theoretical line in which the expression ratio of the Cy3 signal intensity to the Cy5 signal intensity becomes 2:1 or 1:2; and each dotted line is the theoretical line in which the expression ratio of the Cy3 signal intensity to the Cy5 signal intensity becomes 3:1 or 1:3.

As a result, as to the expression ratio of the Cy3 signal intensity to the Cy5 signal intensity, the expression ratio is supposed to be 1:1 for all of the genes. However, as shown in Figure 2, the range of the Cy3/Cy5 ratio deduced to give a 99% convergence in distribution of significant signal spots was calculated to be 1/2.27 to 2.27 based on the (standard deviation of ratio of the Cy3/Cy5 signal intensity). In consideration of the significant difference of expression alteration of 1/2 folds or less or 2 folds or more as generally said, there is a risk that false-positive data may be contained that lead to an erroneous interpretation.

It was found, however, that the variance of spots is notably smaller in Figure 1 as compared to that of Figure 2. In other words, it was found that the labeling method shown in (ii) of Table 3 (Figure 1) was found be a method capable of causing a smaller variance (uniform) of the ratio of labeling signal intensity due to the difference in the fluorescent label compounds as compared to the conventional method shown in (iv) of Table 3 (Figure 2).

Also, as shown in Table 3, it was found that the accuracy becomes higher when the concentration ratio of non-labeled substrate/labeled substrate (non-labeled substrate/labeled substrate = 5/1) was high as compared to that when the concentration ratios of the non-labeled substrate/labeled substrate for Cy3 and Cy5 were set at the same level, and the concentration ratio of the non-labeled substrate/labeled substrate was set low (non-labeled substrate/labeled substrate = 2/1). Moreover, it was found that the accuracy was further increased when the concentration ratios of the non-labeled substrate/labeled substrate were set at different levels for Cy3 and Cy5, and when the concentration ratio of the non-labeled substrate/labeled substrate for Cy3 was 2/1 and the concentration ratio of the non-labeled substrate/labeled substrate for Cy5 was 5/1.

### (2) Comparison with Commercially Available Kit

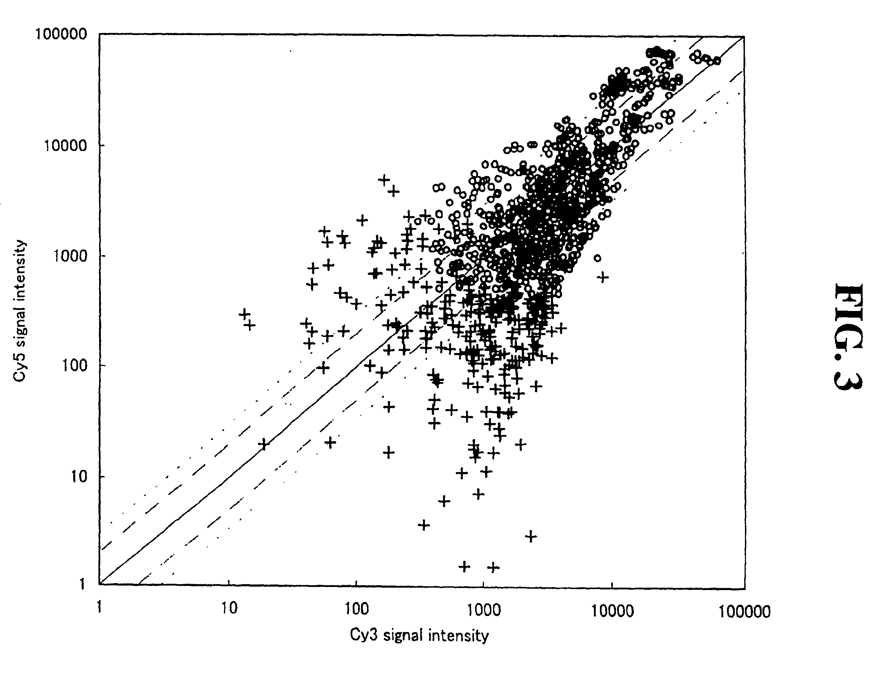

A Cy3-labeled cDNA probe and a Cy5-labeled cDNA probe were prepared with the mRNA of Example 1 as a template using Cyscribe First-strand cDNA Labeling Kit (manufactured by Amersham-Pharmacia), a commercially available kit for preparing CyDye-labeled cDNA probes, in accordance with the instruction manual attached to the kit. The hybridization with the DNA microarray, washing, scanning and analysis were carried out under the same conditions as those described in Example 1. The results of Scatter Plot are shown in Figure 3. Figure 3 is a diagram showing signal intensities obtained when each fluorescent label was used. In the figure, the X-axis represents a Cy3 signal intensity, and the Y-axis represents a Cy5 signal intensity. Also, an open circle (O) represents a spot exhibiting a significant signal intensity, a cross (+) represents a spot exhibiting a non-significant signal intensity, a solid line is a line showing an expression ratio of 1:1, a broken line is a line showing an expression ratio of 2:1 or 1:2, and a dotted line is a line showing an expression ratio of 3:1 or 1:3.

As in the case of the above (1), the expression ratio has to be 1:1 for all of the genes. However, as shown in Figure 3, 57% of the genes showed a difference in expression of 2 folds or more when the Cy3-labeled cDNA probe was used and when the Cy5-labeled cDNA probe was used, and the Cy3/Cy5 ratio which was deduced at a 99% convergence in distribution of significant spots was shown to have a value of 8.5.

It is found, however, that the variance of spots is notably smaller in Figure 1 as compared to that of Figure 3. In other words, it was found that the labeling method shown in (ii) of Table 3 (Figure 1) is a method showing smaller difference in labeling efficiency caused by the difference in the fluorescent label compounds, as compared to that of the labeling method using the commercially available kit (Figure 3).

### Example 3

In order to confirm that the results obtained in Example 1 are phenomena generally found for any sorts of DNA chips without being altered by the properties (degree of background and the like) of particular substrates of DNA chips and DNA microarrays and the binding manner of the DNA with the substrate, the studies were conducted in the same manner as in Example 1 using two kinds of DNA chips having different substrates.

The preparation of the DNA chips was carried out as follows. Concretely, a slide glass into which an activated carboxyl group was introduced was prepared in accordance with the method described in WO 01/02538. Next, approximately 770 kinds of human cancer-related genes listed in Tables 4 to 60 were selected, and primer pairs were designed so that approximately 300 bp regions shown in Tables 4 to 60 can be amplified on the basis of the nucleotide sequences of these genes.

Tables 4 to 60 are tables showing the names of genes and accession numbers (GenBank Accession Numbers) of the cancer-related genes and the specific gene regions selected for each of the genes, wherein the regions are shown by the corresponding numbers of nucleic acids registered in the database.

**Table 4**

| Name of Gene | Accession Number | Selected Gene Region |
|---|---|---|
| SFRS protein kinase 2 | NM_003138 | 2251-2550 |
| matrix metalloproteinase 9(gelatinase B, 92kD gelatinase, 92kD type IV collagenase) | NM_004994 | 1893-2192 |
| fibroblast growth factor 2 (basic) | NM_002006 | 5759-6058 |
| mitogen-activated protein kinase 10 | NM_002753 | 2028-2327 |
| hexabrachion (tenascin C, cytotactin) | NM_002160 | 6557-6856 |
| MAD (mothers against decapentaplegic, Drosophila) homolog 4 | NM_005359 | 1697-1996 |
| Human clone 23878 mRNA sequence | U79251 | 696-397 |
| tumor protein p53 (Li-Fraumeni syndrome) | NM_000546 | 1602-1901 |
| catenin (cadherin-associated protein), alpha 2 | NM_004389 | 2710-3009 |
| cell adhesion molecule with homology to L1CAM (close homologue of L1) | NM_006614 | 6316-6615 |
| contactin 2 (axonal) | NM_005076 | 3922-4221 |
| protein phosphatase 2, regulatory subunit B (B56), alpha isoform | NM_006243 | 2150-2449 |
| v-myc avian myelocytomatosis viral related oncogene, neuroblastoma derived | Y00664 | 1066-1365 |
| mitogen-activated protein kinase 6 | NM_002748 | 2470-2769 |
| serine (or cysteine) proteinase inhibitor, clade B (ovalbumin), member 2 | NM_002575 | 1307-1606 |
| v-yes-1 Yamaguchi sarcoma viral oncogene homolog 1 | NM_005433 | 4214-4513 |

**Table 5**

| Name of Gene | Accession Number | Selected Gene Region |
|---|---|---|
| caspase 3, apoptosis-related cysteine protease | NM_004346 | 1984-2283 |
| matrix metalloproteinase 17 (membrane-inserted) | NM_016155 | 1342-1641 |
| cadherin 13, H-cadherin (heart) | NM_001257 | 3614-3913 |
| eukaryotic translation elongation factor 2 | NM_001961 | 1843-2142 |
| eukaryotic translation elongation factor 2 | NM_001961 | 1842-2141 |
| baculoviral IAP repeat-containing 2 | NM_001166 | 3022-3321 |
| diphtheria toxin receptor (heparin-binding epidermal growth factor-like growth factor) | NM_001945 | 1037-1336 |
| mitogen-activating protein kinase kinase kinase kinase 2 | NM_004579 | 1509-1808 |
| phosphoinositide-3-kinase, regulatory subunit, polypeptide 1 (p85 alpha) | M61906 | 1838-1539 |
| IMP (inosine monophosphate) dehydrogenase 1 | NM_000883 | 2039-2338 |
| ESTs, Highly similar to CAD8_HUMAN CADHERIN-8 PRECURSOR [H.sapiens] | AA552988 | 522-223 |
| lunatic fringe (Drosophila) homolog | U94354 | 1-300 |
| lunatic fringe (Drosophila) homolog | U94354 | 13-312 |
| bone morphogenetic protein 1 | NM_006129 | 2373-2672 |
| bone morphogenetic protein 1 | NM_006129 | 2374-2673 |
| interleukin 13 receptor, alpha 2 | NM_000640 | 836-1135 |
| hepatocyte growth factor(hepapoietin A; scatter factor) | X16323 | 1555-1854 |
| 5' nucleotidase (CD73) | NM_002526 | 3153-3452 |

**Table 6**

| Name of Gene | Accession Number | Selected Gene Region |
|---|---|---|
| TRK-fused gene | NM_006070 | 999-1298 |
| vascular cell adhesion molecule 1 | NM_001078 | 2271-2570 |
| vascular endothelial growth factor C | NM_005429 | 870-1169 |
| G protein-coupled receptor 19 | NM_006143 | 1065-1364 |
| leucyl/cystinyl aminopeptidase | NM_005575 | 2164-2463 |
| Human insulin-like growth factor binding protein 5 (IGFBP5) mRNA | L27560 | 3080-2781 |
| mitogen-activated protein kinase kinase 6 | U39657 | 1283-1582 |
| choline kinase | NM_001277 | 570-869 |
| Human clone 23734 mRNA sequence | U79292 | 1385-1086 |
| p53-induced protein | NM_006034 | 1885-2184 |
| E74-like factor 4(ets domain transcription factor) | NM_001421 | 3231-3530 |
| vascular cell adhesion molecule 1 | NM_001078 | 2271-2570 |
| AXL receptor tyrosine kinase | NM_021913 | 2168-2467 |
| SKI-INTERACTING PROTEIN | NM_012245 | 1186-1485 |
| peroxiredoxin 3 | NM_006793 | 1005-1304 |
| cell division cycle 34 | L22005 | 528-827 |
| general transcription factor IIB | NM_001514 | 333-632 |
| baculoviral IAP repeat-containing 1 | NM_004536 | 4933-5232 |
| excision repair cross-complementing rodent repair deficiency, complementation group 3 (xeroderma pigmentosum group B complementing) | NM_000122 | 1915-2214 |
| manic fringe (Drosophila) homolog | NM_002405 | 1003-1302 |

**Table 7**

| Name of Gene | Accession Number | Selected Gene Region |
|---|---|---|
| platelet-derived growth factor receptor, alpha polypeptide | NM_006206 | 5508-5807 |
| endothelin 3 | NM_000114 | 1586-1885 |
| plasminogen-like | NM_002665 | 225-524 |
| platelet-derived growth factor beta polypeptide (simian sarcoma viral (v-sis) oncogene homolog) | NM_002608 | 2898-3197 |
| platelet-derived growth factor beta polypeptide (simian sarcoma viral (v-sis) oncogene homolog) | NM_002608 | 2897-3196 |
| cyclin E1 | M74093 | 1623-1324 |
| natural killer cell group 7 sequence | NM_005601 | 67-366 |
| caspase 7, apoptosis-related cysteine protease | NM_001227 | 1203-1502 |
| nerve growth factor, beta polypeptide | NM_002506 | 239-538 |
| nerve growth factor, beta polypeptide | NM_002506 | 238-537 |
| exportin 1 (CRM1, yeast, homolog) | NM_003400 | 3542-3841 |
| EphB4 | NM_004444 | 3628-3927 |
| TNF receptor-associated factor 2 | NM_021138 | 1749-2048 |
| small inducible cytokine subfamily A (Cys-Cys), member 16 | NM_004590 | 1196-1495 |
| betaine-homocysteine methyltransferase | NM_001713 | 1353-1652 |
| FBJ murine osteosarcoma viral oncogene homolog B | NM_006732 | 3447-3746 |
| small inducible cytokine subfamily A (Cys-Cys), member 13 | NM_005408 | 470-769 |

**Table 8**

| Name of Gene | Accession Number | Selected Gene Region |
|---|---|---|
| Rho-associated, coiled-coil containing protein kinase 1 | NM_005406 | 1646-1945 |
| GTP-binding protein homologous to Saccharomyces cerevisiae SEC4 | NM_006822 | 1328-1627 |
| lysophospholipase-like | NM_007283 | 802-1101 |
| P450 (cytochrome) oxidoreductase | AF258341 | 1733-2032 |
| glutathione S-transferase A2 | AL109918 | 1472-1771 |
| deoxyribonuclease I-like 3 | NM_004944 | 306-605 |
| growth arrest and DNA-damage-inducible, gamma | NM_006705 | 419-718 |
| growth arrest and DNA-damage-inducible, gamma | NM_006705 | 418-717 |
| plasminogen | NM_000301 | 2380-2679 |
| cell division cycle 27 | NM_001256 | 193-492 |
| caveolin 2 | NM_001233 | 533-832 |
| serine/threonine kinase 4 | NM_006282 | 835-1134 |
| caspase 1, apoptosis-related cysteine protease (interleukin 1, beta, convertase) | NM_001223 | 642-941 |
| excision repair cross-complementing rodent repair deficiency, complementation group 1 (includes overlapping antisense sequence) | NM_001983 | 369-668 |
| signal sequence receptor, alpha (translocon-associated protein alpha) | NM_003144 | 368-667 |
| interferon-related developmental regulator 1 | NM_001550 | 1091-1390 |
| casein kinase 2, alpha 1 polypeptide | NM_001895 | 40-339 |

**Table 9**

| Name of Gene | Accession Number | Selected Gene Region |
|---|---|---|
| collagen, type III, alpha 1 (Ehlers-Danlos syndrome type IV, autosomal dominant) | NM_000090 | 4033-4332 |
| jun B proto-oncogene | NM_002229 | 1229-1528 |
| wingless-type MMTV integration site family member 2 | NM_003391 | 1574-1873 |
| putative chemokine receptor; GTP-binding protein | NM_006018 | 249-548 |
| dihydrofolate reductase | NM_000791 | 455-754 |
| translocated promoter region (to activated MET oncogene) | NM_003292 | 331-630 |
| hyaluronan-mediated motility receptor (RHAMM) | NM_012484 | 1545-1844 |
| intercellular adhesion molecule 2 | NM_000873 | 32-331 |
| peripheral myelin protein 22 | NM_000304 | 1011-1310 |
| protein kinase, DNA-activated, catalytic polypeptide | U47077 | 13130-13429 |
| glutathione S-transferase pi | NM_000852 | 153-452 |
| transforming growth factor, beta 1 | NM_000660 | 1404-1703 |
| Glutamate receptor interacting protein | AJ133439 | 2231-2530 |
| programmed cell death 10 | NM_007217 | 711-1010 |
| mouse double minute 2, human homolog of; p53-binding protein | NM_002392 | 128-427 |
| mouse double minute 2, human homolog of; p53-binding protein | NM_002392 | 129-428 |
| arachidonate 5-lipoxygenase | NM_000698 | 1235-1534 |

**Table 10**

| Name of Gene | Accession Number | Selected Gene Region |
|---|---|---|
| arachidonate 5-lipoxygenase | NM_000698 | 1234-1533 |
| insulin induced gene 1 | NM_005542 | 1393-1692 |
| insulin induced gene 1 | NM_005542 | 1392-1691 |
| guanine monphosphate synthetase | NM_003875 | 1913-2212 |
| chromodomain helicase DNA binding protein 1 | NM_001270 | 5018-5317 |
| glutathione S-transferase theta 1 | NM_000853 | 276-575 |
| collagen, type VI, alpha 3 | NM_004369 | 9819-10118 |
| fibronectin 1 | X02761 | 6690-6989 |
| jagged 1 (Alagille syndrome) | NM_000214 | 5239-5538 |
| KIAA0128 protein; septin 2 | D50918 | 1678-1977 |
| deoxyribonuclease I-like 1 | NM_006730 | 2058-2357 |
| protocadherin 1 (cadherin-like 1) | NM_002587 | 3764-4063 |
| epidermal growth factor receptor pathway substrate 8 | NM_004447 | 3270-3569 |
| wingless-type MMTV integration site family member 2 | NM_003391 | 1574-1873 |
| selectin L (lymphocyte adhesion molecule 1) | NM_000655 | 1451-1750 |
| TEK tyrosine kinase, endothelial (venous malformations, multiple cutaneous and mucosal) | NM_000459 | 3599-3898 |
| TEK tyrosine kinase, endothelial (venous malformations, multiple cutaneous and mucosal) | NM_000459 | 3598-3897 |
| protease, serine, 22 | NM_006025 | 2021-2320 |
| leukemia inhibitory factor (cholinergic differentiation factor) | NM_002309 | 2967-3266 |

**Table 11**

| Name of Gene | Accession Number | Selected Gene Region |
|---|---|---|
| leukemia inhibitory factor (cholinergic differentiation factor) | NM_002309 | 2966-3265 |
| SH3-domain binding protein 2 | AB000462 | 4144-4443 |
| integrin, alpha L(antigen CD11A(p180),lymphocyte function-associated antigen 1;alpha polypeptide) | NM_002209 | 4320-4619 |
| collagen, type XVI, alpha 1 | NM_001856 | 113-412 |
| nerve growth factor receptor (TNFR superfamily, member 16) | NM_002507 | 2719-3018 |
| neuregulin 1 | NM_013957 | 857-1156 |
| GTP-binding protein homologous to Saccharomyces cerevisiae SEC4 | NM_006822 | 468-767 |
| ras homolog gene family, member G (rho G) | NM_001665 | 905-1204 |
| laminin, gamma 1 (formerly LAMB2) | NM_002293 | 833-1132 |
| actin related protein 2/3 complex, subunit 2 (34 kD) | NM_005731 | 640-939 |
| peripheral myelin protein 22 | NM_000304 | 1427-1726 |
| transcription elongation factor A (SII), 1 | NM_006756 | 1748-2047 |
| integrin, alpha 8 | L36531 | 781-1080 |
| adrenergic, beta, receptor kinase 1 | NM_001619 | 1901-2200 |
| adrenergic, beta, receptor kinase 1 | NM_001619 | 1900-2199 |
| protocadherin gamma subfamily C, 3 | NM_002588 | 1731-2030 |
| glia maturation factor, beta | NM_004124 | 3448-3747 |
| mitogen-activated protein kinase 7 | NM_002749 | 2161-2460 |
| mitogen-activated protein kinase 7 | NM_002749 | 2160-2459 |

**Table 12**

| Name of Gene | Accession Number | Selected Gene Region |
|---|---|---|
| purine-rich element binding protein A | NM_005859 | 618-917 |
| purine-rich element binding protein A | NM_005859 | 619-918 |
| catenin (cadherin-associated protein), alpha 2 | NM_004389 | 3410-3709 |
| tumor protein p53-binding protein, 1 | NM_005657 | 1617-1916 |
| activin A receptor type II-like 1 | NM_000020 | 348-647 |
| caspase 4, apoptosis-related cysteine protease | AL050391 | 3835-4134 |
| keratin 14 (epidermolysis bullosa simplex, Dowling-Meara, Koebner) | NM_000526 | 803-1102 |
| myeloid differentiation primary response gene(88) | U70451 | 1851-2150 |
| thrombospondin 2 | NM_003247 | 4969-5268 |
| breast cancer 2, early onset | NM_000059 | 10426-10725 |
| G protein-coupled receptor | NM_006564 | 1471-1770 |
| topoisomerase (DNA) II binding protein | NM_007027 | 4901-5200 |
| baculoviral IAP repeat-containing 3 | AF070674 | 4618-4917 |
| non-metastatic cells 4, protein expressed in | NM_005009 | 570-869 |
| tumor necrosis factor(ligand) superfamily, member10 | NM_003810 | 371-670 |
| CDC6(cell division cycle 6,S.cerevisiae) homolog | NM_001254 | 982-1281 |
| a disintegrin and metalloproteinase domain 9 (meltrin gamma) | NM_003816 | 2763-3062 |
| CD59 antigen p18-20(antigen identified by monoclonal antibodies 16.3A5,EJ16,EJ30,EL32 and G344) | NM_000611 | 803-1102 |

**Table 13**

| Name of Gene | Accession Number | Selected Gene Region |
|---|---|---|
| glycophorin B (includes Ss blood group) | NM_002100 | 88-387 |
| dual-specificity tyrosine-(Y)-phosphorylation regulated kinase 3 | NM_003582 | 1742-2041 |
| transforming growth factor, beta receptor III (betaglycan, 300kD) | NM_003243 | 2704-3003 |
| transforming growth factor, beta receptor III (betaglycan, 300kD) | NM_003243 | 2703-3002 |
| integrin, alpha 1 | X68742 | 3079-2780 |
| tumor protein p53-binding protein, 2 | NM_005426 | 4147-4446 |
| mannosidase, alpha, class 2A, member 1 | NM_002372 | 3502-3801 |
| Rho GTPase activating protein 4 | NM_001666 | 2348-2647 |
| Rho GTPase activating protein 4 | NM_001666 | 2347-2646 |
| Homo sapiens cDNA: FLJ21562 fis, clone COL06420 | AK025215 | 1577-1876 |
| kinase suppressor of ras | U43586 | 748-1047 |
| guanine nucleotide binding protein (G protein), alpha 11 (Gq class) | NM_002067 | 1228-1527 |
| GTP-binding protein | NM_012341 | 1528-1827 |
| B-cell CLL/lymphoma 2 | NM_000633 | 2342-2641 |
| insulin-like growth factor binding protein2(36kD) | NM_000597 | 670-969 |
| GATA-binding protein 6 | NM_005257 | 2138-2437 |
| BCL2-antagonist/killer 1 | NM_001188 | 1616-1915 |
| phospholipase A2, group VII(platelet-activating factor acetylhydrolase, plasma) | NM_005084 | 1036-1335 |

**Table 14**

| Name of Gene | Accession Number | Selected Gene Region |
|---|---|---|
| desmoplakin (DPI, DPII) | NM_004415 | 8024-8323 |
| breast cancer 1, early onset | NM_007295 | 7045-7344 |
| caspase 10, apoptosis-related cysteine protease | NM_001230 | 2967-3266 |
| EphA2 | NM_004431 | 2664-2963 |
| EphA2 | NM_004431 | 2663-2962 |
| tyrosine 3-monooxygenase/tryptophan 5-monooxygenase activation protein, zeta polypeptide | NM_003406 | 2063-2362 |
| inhibitor of growth 1 family, member 1 | NM_005537 | 840-1139 |
| HSPC070 protein | NM_014160 | 2016-2315 |
| RAD52 (S. cerevisiae) homolog | NM_002879 | 2293-2592 |
| insulin-like growth factor 2 (somatomedin A) | X07868 | 819-1118 |
| HMT1(hnRNP methyltransferase,S.cerevisiae)-like 2 | NM_001536 | 595-894 |
| CHK1 (checkpoint, S.pombe) homolog | NM_001274 | 1271-1570 |
| CGI-150 protein | NM_016080 | 1883-2182 |
| vitronectin (serum spreading factor, somatomedin B, complement S-protein) | NM_000638 | 931-1230 |
| vitronectin(serum spreading factor,somatomedin B, complement S-protein) | NM_000638 | 932-1231 |
| cyclin D2 | NM_001759 | 706-1005 |
| dual specificity phosphatase 8 | NM_004420 | 2065-2364 |
| cadherin 1, type 1, E-cadherin (epithelial) | NM_004360 | 4479-4778 |

**Table 15**

| Name of Gene | Accession Number | Selected Gene Region |
|---|---|---|
| cadherin 11, type 2, OB-cadherin (osteoblast) | D21255 | 3033-3332 |
| collagen, type XVII, alpha 1 | AL138761 | 4727-5026 |
| collagen, type XI, alpha 2 | AL031228 | 6121-6420 |
| retinoblastoma-binding protein 6 | NM_006910 | 114-413 |
| guanine nucleotide binding protein-like 1 | NM_005275 | 1411-1710 |
| chorionic gonadotropin, beta polypeptide | NM_000737 | 152-451 |
| chorionic gonadotropin, beta polypeptide | NM_000737 | 151-450 |
| interferon regulatory factor 5 | NM_002200 | 186-485 |
| Human DNA sequence from clone RP4-675C20 on chromosome 1p13.2. Contains the 3' end of the MAN1A2 gene for mannosidase alpha 1A2, a pseudogene similar to predicted fly, worm and yeast genes, ESTs, STSs and GSSs | AL157902 | 2720-3019 |
| cathepsin D (lysosomal aspartyl protease) | NM_001909 | 356-655 |
| bone morphogenetic protein receptor, type II (serine/threonine kinase) | NM_001204 | 2722-3021 |
| v-kit Hardy-Zuckerman 4 feline sarcoma viral oncogene homolog | NM_000222 | 4386-4685 |
| 3-phosphoinositide dependent protein kinase-1 | NM_002613 | 1377-1676 |
| FYN oncogene related to SRC, FGR, YES | Z97989 | 1706-2005 |
| v-kit Hardy-Zuckerman 4 feline sarcoma viral oncogene homolog | NM_000222 | 4386-4685 |
| matrix metalloproteinase 14 (membrane-inserted) | NM_004995 | 2435-2734 |

**Table 16**

| Name of Gene | Accession Number | Selected Gene Region |
|---|---|---|
| CDC7 (cell division cycle 7, S. cerevisiae, homolog)-like 1 | NM_003503 | 2870-3169 |
| allograft inflammatory factor 1 | NM_004847 | 850-1149 |
| mitogen-activated protein kinase kinase 5 | NM_002757 | 1436-1735 |
| cell division cycle 2-like 5 (cholinesterase-related cell division controller) | AJ297709 | 1737-2036 |
| FYN oncogene related to SRC, FGR, YES | Z97989 | 2088-2387 |
| Ser-Thr protein kinase related to the myotonic dystrophy protein kinase | NM_003607 | 2444-2743 |
| tyrosinase (oculocutaneous albinism IA) | NM_000372 | 1665-1964 |
| v-yes-1 Yamaguchi sarcoma viral oncogene homolog1 | NM_005433 | 4215-4514 |
| multifunctional polypeptide similar to SAICAR synthetase and AIR carboxylase | NM_006452 | 492-791 |
| glucuronidase, beta | NM_000181 | 247-546 |
| glucuronidase, beta | NM_000181 | 248-547 |
| FYN oncogene related to SRC, FGR, YES | Z97989 | 1706-2005 |
| hypoxanthine phosphoribosyltransferase 1 (Lesch-Nyhan syndrome) | NM_000194 | 531-830 |
| inhibitor of growth 1 family, member 1 | NM_005537 | 1408-1707 |
| sema domain, immunoglobulin domain (Ig), transmembrane domain (TM) and short cytoplasmic domain, (semaphorin) 4D | NM_006378 | 3258-3557 |
| RAB36, member RAS oncogene family | NM_004914 | 1300-1599 |

**Table 17**

| Name of Gene | Accession Number | Selected Gene Region |
|---|---|---|
| H2A histone family, member L | NM_003512 | 550-849 |
| matrix metalloproteinase 13 (collagenase 3) | NM_002427 | 2193-2492 |
| interferon (alpha, beta and omega) receptor 1 | NM_000629 | 2454-2753 |
| neutral sphingomyelinase (N-SMase) activation associated factor | NM_003580 | 2466-2765 |
| interleukin 15 | NM_000585 | 617-916 |
| interleukin 15 | NM_000585 | 618-917 |
| cyclin-dependent kinase inhibitor 2C (p18, inhibits CDK4) | NM_001262 | 1211-1510 |
| silver (mouse homolog) like | NM_006928 | 1427-1726 |
| dishevelled 3 (homologous to Drosophila dsh) | NM_004423 | 3247-3546 |
| death-associated protein 6 | NM_001350 | 1939-2238 |
| excision repair cross-complementing rodent repair deficiency, complementation group 5 (xeroderma pigmentosum, complementation group G (Cockayne syndrome)) | NM_000123 | 2973-3272 |
| ankyrin 1, erythrocytic | NM_000037 | 7923-8222 |
| lysozyme (renal amyloidosis) | NM_000239 | 1188-1487 |
| Homo sapiens cDNA FLJ11848 fis,clone HEMBA 1006708, weakly similar to HYPOTHETICAL 46.4 KD TRP-ASP REPEATS CONTAINING PROTEIN IN PMCl-TFG2 INTERGENIC REGION | AK021910 | 1129-1428 |
| GTP-binding protein ragB | NM_016656 | 172-471 |

**Table 18**

| Name of Gene | Accession Number | Selected Gene Region |
|---|---|---|
| transcription factor Dp-1 | NM_007111 | 40-339 |
| insulin-like growth factor 2 (somatomedin A) | X07868 | 101-400 |
| glycophorin A (includes MN blood group) | NM_002099 | 399-698 |
| small inducible cytokine subfamily A (Cys-Cys), member 25 | NM_005624 | 64-363 |
| metastasis associated 1 | NM_004689 | 1400-1699 |
| metastasis associated 1 | NM_004689 | 1399-1698 |
| adaptor-related protein complex 1,gamma1 subunit | NM_001128 | 3264-3563 |
| far upstream element (FUSE) binding protein 3 | U69127 | 2280-2579 |
| collagen, type XVIII, alpha 1 | AF018081 | 4732-5031 |
| collagen, type XVIII, alpha 1 | AF018081 | 4731-5030 |
| protein phosphatase 3 (formerly 2B), catalytic subunit, gamma isoform (calcineurin A gamma) | NM_005605 | 282-581 |
| wingless-type MMTV integration site family member 2 | NM_003391 | 1971-2270 |
| ras homolog gene family, member H | NM_004310 | 221-520 |
| E2F transcription factor 3 | NM_001949 | 4003-4302 |
| v-Ki-ras2 Kirsten rat sarcoma 2 viral oncogene homolog | NM_004985 | 674-973 |
| replication factor C (activator 1) 4 (37kD) | NM_002916 | 1090-1389 |
| CASP8 and FADD-like apoptosis regulator | Y14039 | 557-856 |

**Table 19**

| Name of Gene | Accession Number | Selected Gene Region |
|---|---|---|
| jun B proto-oncogene | NM_002229 | 1229-1528 |
| jun B proto-oncogene | NM_002229 | 1228-1527 |
| active BCR-related gene | NM_021962 | 4670-4969 |
| active BCR-related gene | NM_021962 | 4669-4968 |
| retinoic acid- and interferon-inducible protein (58kD) | NM_012420 | 2990-3289 |
| CGI-39 protein; cell death-regulatory protein GRIM19 | NM_015965 | 217-516 |
| chromogranin B (secretogranin 1) | NM_001819 | 1958-2257 |
| phosphatase and tensin homolog (mutated in multiple advanced cancers 1) | NM_000314 | 2666-2965 |
| fibroblast growth factor 2 (basic) | NM_002006 | 3424-3723 |
| matrix metalloproteinase 3 (stromelysin 1, progelatinase) | NM_002422 | 860-1159 |
| interleukin 1, beta | NM_000576 | 1048-1347 |
| Notch (Drosophila) homolog 3 | NM_000435 | 3583-3882 |
| Notch (Drosophila) homolog 3 | NM_000435 | 3582-3881 |
| epidermal growth factor receptor (avian erythroblastic leukemia viral (v-erb-b) oncogene homolog) | NM_005228 | 5063-5362 |
| wingless-type MMTV integration site family, member 5A | NM_003392 | 1965-2264 |
| cadherin 2, type 1, N-cadherin (neuronal) | S42303 | 3171-3470 |

**Table 20**

| Name of Gene | Accession Number | Selected Gene Region |
|---|---|---|
| cadherin 2, type 1, N-cadherin (neuronal) | S42303 | 3170-3469 |
| keratin 13 | NM_002274 | 1286-1585 |
| elastase 3B | NM_007352 | 184-483 |
| CD44 antigen (homing function and Indian blood group system) | AJ251595 | 2468-2767 |
| mitogen-activated protein kinase kinase kinase 8 | NM_005204 | 55-354 |
| mitogen-activated protein kinase kinase kinase 8 | NM_005204 | 54-353 |
| integrin, beta 8 | NM_002214 | 2753-3052 |
| keratin 13 | NM_002274 | 1292-1591 |
| RAB2, member RAS oncogene family | NM_002865 | 590-889 |
| RAB2, member RAS oncogene family | NM_002865 | 589-888 |
| B-cell CLL/lymphoma 2 | NM_000633 | 5307-5606 |
| mitogen-activated protein kinase kinase kinase14 | NM_003954 | 3516-3815 |
| platelet-derived growth factor beta polypeptide (simian sarcoma viral (v-sis) oncogene homolog) | NM_002608 | 2898-3197 |
| platelet-derived growth factor beta polypeptide (simian sarcoma viral (v-sis) oncogene homolog) | NM_002608 | 2897-3196 |
| uridine monophosphate kinase | NM_012474 | 165-464 |
| microsomal glutathione S-transferase 2 | NM_002413 | 223-522 |
| Fas-activated serine/threonine kinase | NM_006712 | 1098-1397 |
| Fas-activated serine/threonine kinase | NM-006712 | 1099-1398 |
| EphB2 | AF025304 | 3508-3807 |

**Table 21**

| Name of Gene | Accession Number | Selected Gene Region |
|---|---|---|
| lymphotoxin alpha (TNF superfamily, member 1) | NM_000595 | 845-1144 |
| lymphotoxin alpha (TNF superfamily, member 1) | NM_000595 | 846-1145 |
| general transcription factor IIH, polypeptide 2 (44kD subunit) | NM_001515 | 764-1063 |
| cathepsin L | NM_001912 | 1063-1362 |
| v-akt murine thymoma viral oncogene homolog 1 | NM_005163 | 562-861 |
| guanylate binding protein 2,interferon-inducible | NM_004120 | 696-995 |
| guanylate binding protein 2,interferon-inducible | NM_004120 | 697-996 |
| angiogenin, ribonuclease, RNase A family, 5 | NM_001145 | 95-394 |
| eukaryotic translation initiation factor 2, subunit 2 (beta, 38kD) | NM_003908 | 857-1156 |
| retinoblastoma 1 (including osteosarcoma) | NM_000321 | 3892-4191 |
| mitogen-activated protein kinase 1 | AL157438 | 1097-798 |
| tumor necrosis factor, alpha-induced protein 6 | NM_007115 | 447-746 |
| neuronal Shc | NM_016848 | 1106-1405 |
| heterogeneous nuclear ribonucleoprotein H1 (H) | NM_005520 | 1459-1758 |
| v-jun avian sarcoma virus 17 oncogene homolog | NM_002228 | 2823-3122 |
| transmembrane trafficking protein | NM_006827 | 125-424 |
| BCL2/adenovirus E1B 19kD-interacting protein 3 | NM_004052 | 468-767 |
| ataxia telangiectasia mutated (includes complementation groups A, C and D) | U82828 | 12746-13045 |
| protein kinase, cAMP-dependent, catalytic, beta | NM_002731 | 2330-2629 |
| IKK-related kinase epsilon; inducible IkappaB kinase | NM_014002 | 2920-3219 |

**Table 22**

| Name of Gene | Accession Number | Selected Gene Region |
|---|---|---|
| fibroblast growth factor 7 (keratinocyte growth factor) | NM_002009 | 1451-1750 |
| primase, polypeptide 1 (49kD) | NM_000946 | 812-1111 |
| envoplakin | NM_001988 | 6100-6399 |
| envoplakin | NM_001988 | 6099-6398 |
| Human DNA sequence from clone RP11-560A15 on chromosome 20 Contains part of a novel gene, the 3' part of the BMP7 (bone morphogenetic protein 7 (osteogenic protein 1)) gene, ESTs, STSs, GSSs and a CpG island | AL157414 | 2443-2742 |
| topoisomerase (DNA) II alpha (170kD) | NM_001067 | 3593-3892 |
| wee1+ (S. pombe) homolog | X62048 | 2495-2794 |
| caveolin 1, caveolae protein, 22kD | NM_001753 | 460-759 |
| integrin, alpha 7 | NM_002206 | 3585-3884 |
| integrin, alpha 7 | NM_002206 | 3584-3883 |
| reticulon 3 | NM_006054 | 1903-2202 |
| 30 kDa protein | NM_018447 | 378-677 |
| neutral sphingomyelinase (N-SMase) activation associated factor | NM_003580 | 993-1292 |
| cell division cycle 25C | NM_001790 | 1288-1587 |
| dishevelled 3 (homologous to Drosophila dsh) | NM_004423 | 4927-5226 |

**Table 23**

| Name of Gene | Accession Number | Selected Gene Region |
|---|---|---|
| ras homolog gene family, member E | NM_005168 | 42-341 |
| serine(or cysteine)proteinase inhibitor,clade A (alpha-1 antiproteinase, antitrypsin), member 5 | NM_000624 | 344-643 |
| cell growth regulatory with ring finger domain | NM_006568 | 503-802 |
| pro-platelet basic protein (includes platelet basic protein, beta-thromboglobulin, connective tissue-activating peptide III, neutrophil-activating peptide-2) | NM_002704 | 252-551 |
| microtubule-associated protein, RP/EB family, member 1 | NM_012325 | 1469-1768 |
| baculoviral IAP repeat-containing 3 | AF070674 | 2981-3280 |
| caspase 3, apoptosis-related cysteine protease | NM_004346 | 647-946 |
| H2B histone family, member Q | NM_003528 | 447-746 |
| centromere protein F (350/400kD, mitosin) | NM_005196 | 9796-10095 |
| platelet-derived growth factor alpha polypeptide | NM_002607 | 1742-2041 |
| H.sapiens mRNA for hcgVIII protein | X92110 | 515-216 |
| delta-like homolog (Drosophila) | NM_003836 | 726-1025 |
| delta-like homolog (Drosophila) | NM_003836 | 727-1026 |
| tumor necrosis factor(TNF superfamily, member 2) | NM_000594 | 1122-1421 |
| baculoviral IAP repeat-containing 2 | NM_001166 | 1489-1788 |
| retinoid X receptor, alpha | NM_002957 | 1197-1496 |
| cell growth regulatory with ring finger domain | NM_006568 | 503-802 |

**Table 24**

| Name of Gene | Accession Number | Selected Gene Region |
|---|---|---|
| kinase insert domain receptor(a type III receptor tyrosine kinase) | NM_002253 | 4800-5099 |
| KIAA0001 gene product; putative G-protein-coupled receptor; G protein coupled receptor for UDP-glucose | NM_014879 | 1670-1969 |
| ligase III, DNA, ATP-dependent | NM_013975 | 2981-3280 |
| ligase III, DNA, ATP-dependent | NM_013975 | 2982-3281 |
| matrix metalloproteinase 7 (matrilysin, uterine) | Z11887 | 365-664 |
| RAB32, member RAS oncogene family | NM_006834 | 455-754 |
| transforming growth factor, beta 3 | NM_003239 | 1970-2269 |
| sema domain, immunoglobulin domain (Ig), short basic domain, secreted, (semaphorin) 3C | NM_006379 | 3212-3511 |
| integrin beta 3 binding protein(beta3-endonexin) | NM_014288 | 580-879 |
| tissue inhibitor of metalloproteinase 3 (Sorsby fundus dystrophy, pseudoinflammatory) | NM_000362 | 2599-2898 |
| chondroitin sulfate proteoglycan 2 (versican) | U16306 | 10927-11226 |
| CD58 antigen, (lymphocyte function-associated antigen 3) | NM_001779 | 229-528 |
| glycoprotein A repetitions predominant | NM_005512 | 3656-3955 |
| glycoprotein A repetitions predominant | NM_005512 | 3655-3954 |
| cadherin 11, type 2, OB-cadherin (osteoblast) | D21255 | 2919-3218 |
| STAT induced STAT inhibitor-2 | NM_003877 | 509-808 |

**Table 25**

| Name of Gene | Accession Number | Selected Gene Region |
|---|---|---|
| collagen, type IV, alpha 1 | NM_001845 | 2609-2908 |
| interleukin 1, beta | NM_000576 | 218-517 |
| collagen, type XVII, alpha 1 | AL138761 | 5168-5467 |
| laminin, beta 2 (laminin S) | NM_002292 | 5273-5572 |
| histone deacetylase 2 | NM_001527 | 1556-1855 |
| retinoblastoma-binding protein 1 | NM_002892 | 4077-4376 |
| Wilms tumor 1 | X51630 | 1849-2148 |
| monokine induced by gamma interferon | NM_002416 | 2054-2353 |
| hemopoietic cell kinase | NM_002110 | 1499-1798 |
| hemopoietic cell kinase | NM_002110 | 1498-1797 |
| syndecan 4 (amphiglycan, ryudocan) | NM_002999 | 2316-2615 |
| mitogen-activated protein kinase kinase kinase 5 | NM_005923 | 4005-4304 |
| carcinoembryonic antigen-related cell adhesion molecule 6(non-specific cross reacting antigen) | M18216 | 1941-2240 |
| signal sequence receptor, alpha (translocon-associated protein alpha) | NM_003144 | 2229-2528 |
| ectodermal-neural cortex (with BTB-like domain) | NM_003633 | 1680-1979 |
| ectodermal-neural cortex (with BTB-like domain) | NM_003633 | 1681-1980 |
| protein phosphatase 2, regulatory subunit B (B56), beta isoform | NM_006244 | 2006-2305 |
| peroxisome proliferative activated receptor,gamma | NM_005037 | 744-1043 |
| cadherin 17, LI cadherin (liver-intestine) | NM_004063 | 2283-2582 |
| syndecan 1 | NM_002997 | 1547-1846 |

**Table 26**

| Name of Gene | Accession Number | Selected Gene Region |
|---|---|---|
| c-src tyrosine kinase | NM_004383 | 2049-2348 |
| guanine nucleotide binding protein (G protein), alpha inhibiting activity polypeptide 2 | NM_002070 | 1453-1752 |
| protein kinase, AMP-activated, gamma 1 non-catalytic subunit | NM_002733 | 697-996 |
| interleukin 8 | NM_000584 | 760-1059 |
| phosphatidylinositol glycan, class F | NM_002643 | 509-808 |
| phosphatidylinositol glycan, class F | NM_002643 | 508-807 |
| protein kinase C-like 2 | NM_006256 | 2606-2905 |
| tousled-like kinase 1 | NM_012290 | 3183-3482 |
| TNF receptor-associated factor 5 | NM_004619 | 3668-3967 |
| growth factor receptor-bound protein 10 | D86962 | 4997-5296 |
| cytokeratin 20 | X73502 | 871-1170 |
| placental growth factor, vascular endothelial growth factor-related protein | NM_002632 | 1095-1394 |
| placental growth factor, vascular endothelial growth factor-related protein | NM_002632 | 1096-1395 |
| matrix metalloproteinase 1 (interstitial collagenase) | NM_002421 | 711-1010 |
| Tat-interacting protein (30kD) | NM_006410 | 424-723 |
| adenylate cyclase 6 | NM_015270 | 6138-6437 |
| integrin-linked kinase | NM_004517 | 952-1251 |
| keratin 5 (epidermolysis bullosa simplex, Dowling-Meara/Kobner/Weber-Cockayne types) | NM_000424 | 2150-2449 |

**Table 27**

| Name of Gene | Accession Number | Selected Gene Region |
|---|---|---|
| patched (Drosophila) homolog | U43148 | 4363-4662 |
| IGF-II mRNA-binding protein 3 | NM_006547 | 2860-3159 |
| TRAP interacting protein | NM_005879 | 1359-1658 |
| TRAP interacting protein | NM_005879 | 1358-1657 |
| mutS (E. coli) homolog 2 (colon cancer, nonpolyposis type 1) | NM_000251 | 2307-2606 |
| mutS (E. coli) homolog 2 (colon cancer, nonpolyposis type 1) | NM_000251 | 2306-2605 |
| CD27-binding (Siva) protein | NM_006427 | 69-368 |
| CD27-binding (Siva) protein | NM_006427 | 68-367 |
| eukaryotic translation initiation factor 4E | NM_001968 | 355-654 |
| topoisomerase (DNA) I | NM_003286 | 3019-3318 |
| brain-derived neurotrophic factor | NM_001709 | 882-1181 |
| heat shock transcription factor 2 | NM_004506 | 1646-1945 |
| Gardner-Rasheed feline sarcoma viral (v-fgr) oncogene homolog | NM_005248 | 1911-2210 |
| fragile histidine triad gene | NM_002012 | 257-556 |
| multiple endocrine neoplasia I | NM_000244 | 2411-2710 |
| v-rel avian reticuloendotheliosis viral oncogene homolog B (nuclear factor of kappa light polypeptide gene enhancer in B-cells 3) | NM_006509 | 1216-1515 |
| osteoclast stimulating factor 1 | NM_012383 | 28-327 |

**Table 28**

| Name of Gene | Accession Number | Selected Gene Region |
|---|---|---|
| glutaredoxin (thioltransferase) | NM_002064 | 1-300 |
| glutaredoxin (thioltransferase) | NM_002064 | 2-301 |
| xeroderma pigmentosum, complementation group C | NM_004628 | 3067-3366 |
| myxovirus (influenza) resistance 2, homolog of murine | NM_002463 | 2096-2395 |
| nuclear protein, ataxia-telangiectasia locus | NM_002519 | 4610-4909 |
| RAP1A, member of RAS oncogene family | NM_002884 | 1023-1322 |
| suppression of tumorigenicity 16 (melanoma differentiation) | NM_006850 | 1177-1476 |
| signal transducer and activator of transcription 5B | NM_012448 | 2327-2626 |
| MAP-kinase activating death domain | NM_003682 | 5354-5653 |
| CDC-like kinase 2 | NM_003993 | 1334-1633 |
| CDC-like kinase 2 | NM_003993 | 1333-1632 |
| CD44 antigen (homing function and Indian blood group system) | AJ251595 | 1772-2071 |
| PCTAIRE protein kinase 1 | NM_006201 | 1263-1562 |
| PCTAIRE protein kinase 1 | NM_006201 | 1262-1561 |
| plasminogen activator, urokinase | NM_002658 | 1929-2228 |
| tumor necrosis factor receptor superfamily, member 6 | NM_000043 | 2212-2511 |
| interferon-stimulated transcription factor 3, gamma (48kD) | NM_006084 | 1061-1360 |

**Table 29**

| Name of Gene | Accession Number | Selected Gene Region |
|---|---|---|
| chromosome condensation 1 | NM_001269 | 1853-2152 |
| CD8 antigen, beta polypeptide 1 (p37) | NM_004931 | 155-454 |
| B-cell CLL/lymphoma 7B | NM_001707 | 686-985 |
| G protein-coupled receptor kinase 6 | NM_002082 | 1483-1782 |
| malate dehydrogenase 1, NAD (soluble) | NM_005917 | 713-1012 |
| estrogen receptor 1 | NM_000125 | 6059-6358 |
| PCTAIRE protein kinase 3 | AL161977 | 2041-1742 |
| transcription factor AP-2 gamma (activating enhancer-binding protein 2 gamma) | U85658 | 1898-2197 |
| signal transducer and activator of transcription 3 (acute-phase response factor) | NM_003150 | 2380-2679 |
| mitogen-activated protein kinase kinase kinase 4 | NM_005922 | 4978-5277 |
| CD9 antigen (p24) | NM_001769 | 556-855 |
| lymphocyte-specific protein tyrosine kinase | NM_005356 | 1361-1660 |
| mutS (E. coli) homolog 3 | NM_002439 | 635-934 |
| mutS (E. coli) homolog 3 | NM_002439 | 634-933 |
| cadherin 6, type 2, K-cadherin (fetal kidney) | NM_004932 | 2624-2923 |
| membrane-associated tyrosine- and threonine-specific cdc2-inhibitory kinase | NM_004203 | 1594-1893 |
| interferon regulatory factor 1 | NM_002198 | 1558-1857 |
| branched chain keto acid dehydrogenase E1,alpha polypeptide (maple syrup urine disease) | NM_000709 | 852-1151 |
| mitogen-activated protein kinase 4 | NM_002747 | 3570-3869 |

**Table 30**

| Name of Gene | Accession Number | Selected Gene Region |
|---|---|---|
| ESTs, Highly similar to beta-1,3-N-acetylglucosaminyltransferase [H.sapiens] | BF568451 | 74-373 |
| cyclin-dependent kinase 5 | NM_004935 | 609-908 |
| hsp70-interacting protein | NM_012267 | 1185-1484 |
| polo (Drosophia)-like kinase | NM_005030 | 1384-1683 |
| ninjurin 1 | NM_004148 | 937-1236 |
| thyroid receptor interacting protein 15 | NM_004236 | 1102-1401 |
| LPS-induced TNF-alpha factor | NM_004862 | 458-757 |
| fibroblast growth factor receptor 3 (achondroplasia, thanatophoric dwarfism) | M64347 | 3202-3501 |
| c-mer proto-oncogene tyrosine kinase | NM_006343 | 2993-3292 |
| carcinoembryonic antigen-related cell adhesion molecule 1 (biliary glycoprotein) | X16354 | 3053-3352 |
| damage-specific DNA binding protein 2 (48kD) | NM_000107 | 492-791 |
| damage-specific DNA binding protein 2 (48kD) | NM_000107 | 491-790 |
| insulin-like growth factor binding protein 6 | NM_002178 | 589-888 |
| Ras-related GTP-binding protein | NM_006570 | 1051-1350 |
| small inducible cytokine B subfamily(Cys-X-Cys motif), member 13 (B-cell chemoattractant) | NM_006419 | 404-703 |
| autocrine motility factor receptor | AF124145 | 2066-2365 |
| thrombopoietin(myeloproliferative leukemia virus oncogene ligand, megakaryocyte growth and development factor) | NM_000460 | 1205-1504 |

**Table 31**

| Name of Gene | Accession Number | Selected Gene Region |
|---|---|---|
| prostaglandin E synthase | AF217965 | 1519-1818 |
| integrin, alpha M (complement component receptor 3, alpha; also known as CD11b (p170), macrophage antigen alpha polypeptide) | NM_000632 | 3572-3871 |
| integrin, alpha M (complement component receptor 3, alpha; also known as CD11b (p170), macrophage antigen alpha polypeptide) | NM_000632 | 3571-3870 |
| met proto-oncogene (hepatocyte growth factor receptor) | NM_000245 | 790-1089 |
| 37 kDa leucine-rich repeat (LRR) protein | NM_005824 | 1402-1701 |
| ubiquitin specific protease 9, X chromosome (Drosophila fat facets related) | NM_004652 | 7854-8153 |
| uridine monophosphate synthetase (orotate phosphoribosyl transferase and orotidine-5'-decarboxylase) | NM_000373 | 601-900 |
| uridine monophosphate synthetase (orotate phosphoribosyl transferase and orotidine-5'-decarboxylase) | NM_000373 | 600-899 |
| zona pellucida glycoprotein 3A (sperm receptor) | NM_007155 | 880-1179 |
| phosphorylase kinase, beta | NM_000293 | 3265-3564 |

**Table 32**

| Name of Gene | Accession Number | Selected Gene Region |
|---|---|---|
| prefoldin 5 | NM_002624 | 542-841 |
| reticulon 3 | NM_006054 | 791-1090 |
| dual-specificity tyrosine-(Y)-phosphorylation regulated kinase 2 | NM_006482 | 1393-1692 |
| interleukin 18 receptor 1 | NM_003855 | 2830-3129 |
| villin 2 (ezrin) | NM_003379 | 2744-3043 |
| CDC16 (cell division cycle 16, S. cerevisiae, homolog) | NM_003903 | 1735-2034 |
| integrin, alpha 3 (antigen CD49C, alpha 3 subunit of VLA-3 receptor) | NM_002204 | 3756-4055 |
| integrin, alpha 3 (antigen CD49C, alpha 3 subunit of VLA-3 receptor) | NM_002204 | 3755-4054 |
| retinoic acid receptor, beta | NM_000965 | 1997-2296 |
| non-metastatic cells 2, protein (NM23B) expressed in | NM_002512 | 342-641 |
| dystroglycan 1 (dystrophin-associated glycoprotein 1) | NM_004393 | 4389-4688 |
| Ras homolog enriched in brain 2 | NM_005614 | 688-987 |
| tyrosine kinase 2 | NM_003331 | 3583-3882 |
| branched chain aminotransferase 2, mitochondrial | NM_001190 | 1207-1506 |
| ephrin-B1 | NM_004429 | 2213-2512 |
| thrombospondin 4 | NM_003248 | 2709-3008 |

**Table 33**

| Name of Gene | Accession Number | Selected Gene Region |
|---|---|---|
| Human DNA sequence from clone 223H9 on chromosome 22q12.3-13.2. Contains the gene for TOB4 (BTG1 family protein) and the gene for ortholog of A. thaliana F23F1.8. Contains ESTs, STSs, GSSs and four putative CpG islands | AL008582 | 80-379 |
| GTP-binding protein overexpressed in skeletal muscle | NM_005261 | 1320-1619 |
| bone morphogenetic protein 6 | NM_001718 | 2347-2646 |
| dihydrofolate reductase | NM_000791 | 2728-3027 |
| E2F transcription factor 1 | NM_005225 | 1862-2161 |
| mitogen-activated protein kinase kinase 2 | L11285 | 1355-1056 |
| glutathione transferase zeta 1 (maleylacetoacetate isomerase) | NM_001513 | 499-798 |
| nucleoside phosphorylase | NM_000270 | 857-1156 |
| collagen, type IV, alpha 2 | AK025912 | 2644-2345 |
| paxillin | NM_002859 | 2855-3154 |
| Fanconi anemia, complementation group G | NM_004629 | 2228-2527 |
| CD34 antigen | NM_001773 | 1945-2244 |
| CD34 antigen | NM_001773 | 1944-2243 |
| integrin, beta 5 | NM_002213 | 2588-2887 |
| dihydropyrimidine dehydrogenase | NM_000110 | 3815-4114 |
| Rho GTPase activating protein 1 | U02570 | 3045-3344 |
| Rho GTPase activating protein 1 | U02570 | 3044-3343 |

**Table 34**

| Name of Gene | Accession Number | Selected Gene Region |
|---|---|---|
| v-rel avian reticuloendotheliosis viral oncogene homolog A (nuclear factor of kappa light polypeptide gene enhancer in B-cells 3 (p65)) | NM_021975 | 1998-2297 |
| v-rel avian reticuloendotheliosis viral oncogene homolog A (nuclear factor of kappa light polypeptide gene enhancer in B-cells 3 (p65)) | NM_021975 | 1997-2296 |
| protocadherin 1 (cadherin-like 1) | NM_002587 | 3740-4039 |
| mitogen-activated protein kinase 14 | NM_001315 | 3179-3478 |
| keratin 10 (epidermolytic hyperkeratosis; keratosis palmaris et plantaris) | NM_000421 | 1630-1929 |
| serine/threonine kinase 24(Ste20, yeast homolog) | NM_003576 | 1361-1660 |
| 5T4 oncofetal trophoblast glycoprotein | NM_006670 | 1259-1558 |
| formyl peptide receptor 1 | NM_002029 | 581-880 |
| cadherin 3, type 1, P-cadherin (placental) | NM_001793 | 2857-3156 |
| integrin, alpha E (antigen CD103, human mucosal lymphocyte antigen 1; alpha polypeptide) | NM_002208 | 3413-3712 |
| deleted in lymphocytic leukemia, 1 | NM_005887 | 213-512 |
| protein tyrosine phosphatase,non-receptor type 2 | NM_002828 | 987-1286 |
| retinoblastoma-binding protein 4 | NM_005610 | 1329-1628 |
| Fas (TNFRSF6)-associated via death domain | NM_003824 | 1343-1642 |
| glutathione-S-transferase like; glutathione transferase omega | NM_004832 | 408-707 |

**Table 35**

| Name of Gene | Accession Number | Selected Gene Region |
|---|---|---|
| neural precursor cell expressed, developmentally down-regulated 4 | D42055 | 5168-5467 |
| catenin (cadherin-associated protein), beta 1 (88kD) | NM_001904 | 2635-2934 |
| catenin (cadherin-associated protein), beta 1 (88kD) | NM_001904 | 2634-2933 |
| suppression of tumorigenicity 5 | NM_005418 | 3977-4276 |
| ATP-binding cassette, sub-family C (CFTR/MRP), member 3 | NM_020038 | 5065-5364 |
| adenylosuccinate synthase | AK025514 | 1199-900 |
| caspase 8, apoptosis-related cysteine protease | X98172 | 2545-2844 |
| caspase 8, apoptosis-related cysteine protease | X98172 | 2546-2845 |
| v-erb-b2 avian erythroblastic leukemia viral oncogene homolog 2 (neuro/glioblastoma derived oncogene homolog) | NM_004448 | 3790-4089 |
| tyrosine kinase with immunoglobulin and epidermal growth factor homology domains | NM_005424 | 3388-3687 |
| fibroblast growth factor receptor 4 | NM_002011 | 2400-2699 |
| stromal cell-derived factor 1 | NM_000609 | 2959-3258 |
| matrix metalloproteinase 15 (membrane-inserted) | NM_002428 | 3125-3424 |
| A kinase (PRKA) anchor protein (gravin) 12 | NM_005100 | 6230-6529 |
| interferon gamma receptor 2 (interferon gamma transducer 1) | NM_005534 | 1475-1774 |

**Table 36**

| Name of Gene | Accession Number | Selected Gene Region |
|---|---|---|
| signal sequence receptor, alpha (translocon-associated protein alpha) | NM_003144 | 610-909 |
| GS2 gene | U03886 | 465-764 |
| ubiquitin-conjugating enzyme E2M (homologous to yeast UBC12) | NM_003969 | 288-587 |
| cell division cycle 25B | NM_021874 | 2670-2969 |
| Human DNA sequence from clone RP4-675C20 on chromosome 1p13.2. Contains the 3' end of the MAN1A2 gene for mannosidase alpha 1A2, a pseudogene similar to predicted fly, worm and yeast genes, ESTs, STSs and GSSs | AL157902 | 5521-5820 |
| dishevelled 2 (homologous to Drosophila dsh) | NM_004422 | 287-586 |
| dishevelled 2 (homologous to Drosophila dsh) | NM_004422 | 286-585 |
| ataxia telangiectasia and Rad3 related | NM_001184 | 7656-7955 |
| tumor necrosis factor receptor superfamily, member 10b | AF016266 | 1359-1658 |
| cullin 2 | NM_003591 | 2150-2449 |
| dual-specificity tyrosine-(Y)-phosphorylation regulated kinase 4 | AF263541 | 1421-1720 |
| H2A histone family, member L | NM_003512 | 1192-1491 |
| proliferating cell nuclear antigen | NM_002592 | 27-326 |
| nuclear factor of kappa light polypeptide gene enhancer in B-cells 1 (p105) | NM_003998 | 3264-3563 |

**Table 37**

| Name of Gene | Accession Number | Selected Gene Region |
|---|---|---|
| thioredoxin reductase 1 | NM_003330 | 3103-3402 |
| cyclin H | NM_001239 | 418-717 |
| superoxide dismutase 3, extracellular | NM_003102 | 1586-1885 |
| guanine nucleotide binding protein 10 | NM_004125 | 316-615 |
| testis-specific kinase 1 | NM_006285 | 2124-2423 |
| retinoblastoma-binding protein 2 | NM_005056 | 5907-6206 |
| growth arrest and DNA-damage-inducible 34 | NM_014330 | 1843-2142 |
| growth arrest and DNA-damage-inducible 34 | NM_014330 | 1844-2143 |
| growth arrest-specific 6 | NM_000820 | 1591-1890 |
| dystrophin (muscular dystrophy, Duchenne and Becker types), includes DXS142, DXS164, DXS206, DXS230, DXS239, DXS268, DXS269, DXS270, DXS272 | NM_004010 | 12818-13117 |
| adducing 3 (gamma) | NM_016824 | 2352-2651 |
| ornithine decarboxylase 1 | NM_002539 | 1224-1523 |
| baculoviral IAP repeat-containing 5 (survivin) | NM_001168 | 965-1264 |
| baculoviral IAP repeat-containing 5 (survivin) | NM_001168 | 964-1263 |
| bone morphogenetic protein 4 | NM_001202 | 1621-1920 |
| bone morphogenetic protein 4 | NM_001202 | 1620-1919 |
| BCL2-like 2 | D87461 | 2638-2937 |
| ribosomal protein S5 | NM_001009 | 1-300 |
| interleukin16(lymphocyte chemoattractant factor) | M90391 | 1904-2203 |

**Table 38**

| Name of Gene | Accession Number | Selected Gene Region |
|---|---|---|
| plasminogen activator, urokinase receptor | NM_002659 | 899-1198 |
| defender against cell death 1 | NM_001344 | 15-314 |
| pleckstrin homology,Sec7 and coiled/coil domains 2-like | U59752 | 672-971 |
| keratin 19 | NM_002276 | 645-944 |
| deoxythymidylate kinase (thymidylate kinase) | NM_012145 | 593-892 |
| ephrin-B3 | NM_001406 | 2694-2993 |
| integrin, beta 4 | NM_000213 | 4125-4424 |
| Sjogren's syndrome/scleroderma autoantigen 1 | NM_006396 | 40-339 |
| phosphoinositide-3-kinase, class 3 | NM_002647 | 2610-2909 |
| death-associated protein | NM_004394 | 1309-1608 |
| absent in melanoma 2 | NM_004833 | 113-412 |
| mucin 1, transmembrane | J05582 | 3467-3766 |
| mucin 1, transmembrane | J05582 | 3466-3765 |
| signal transducer and activator of transcription 1, 91kD | NM_007315 | 3102-3401 |
| alpha-2-macroglobulin | NM_000014 | 4172-4471 |
| aldolase A, fructose-bisphosphate | NM_000034 | 300-599 |
| aldolase A, fructose-bisphosphate | NM_000034 | 299-598 |
| X-ray repair complementing defective repair in Chinese hamster cells 5 (double-strand-break rejoining; Ku autoantigen, 80kD) | NM_021141 | 1721-2020 |

**Table 39**

| Name of Gene | Accession Number | Selected Gene Region |
|---|---|---|
| X-ray repair complementing defective repair in Chinese hamster cells 5 (double-strand-break rejoining; Ku autoantigen, 80kD) | NM_021141 | 1720-2019 |
| serine/threonine kinase 3 (Ste20, yeast homolog) | NM_006281 | 2080-2379 |
| thrombospondin 1 | NM_003246 | 1780-2079 |
| low density lipoprotein-related protein 1 (alpha-2-macroglobulin receptor) | NM_002332 | 13836-14135 |
| B-cell associated protein | NM_007273 | 678-977 |
| ankyrin 1, erythrocytic | NM_000037 | 6276-6575 |
| myeloid leukemia factor 2 | NM_005439 | 1083-1382 |
| claudin 10 | NM_006984 | 513-812 |
| CDC37 (cell division cycle 37, S. cerevisiae, homolog) | NM_007065 | 1087-1386 |
| CDC37 (cell division cycle 37, S. cerevisiae, homolog) | NM_007065 | 1088-1387 |
| CDC28 protein kinase 1 | NM_001826 | 80-379 |
| glutathione peroxidase 1 | NM_000581 | 631-930 |
| adenosine monophosphate deaminase 2 (isoform L) | AK025706 | 3707-3408 |
| thyroid hormone receptor interactor 6 | AF000974 | 904-1203 |
| F-box only protein 9 | AL137520 | 1571-1870 |
| lymphotoxin beta receptor (TNFR superfamily, member 3 | NM_002342 | 1563-1862 |
| lymphotoxin beta receptor (TNFR superfamily, member 3 | NM_002342 | 1564-1863 |

**Table 40**

| Name of Gene | Accession Number | Selected Gene Region |
|---|---|---|
| adenomatosis polyposis coli | NM_000038 | 8168-8467 |
| glycophorin B (includes Ss blood group) | NM_002100 | 88-387 |
| mannosidase, alpha, class 1A, member 2 | NM_006699 | 577-876 |
| KiSS-1 metastasis-suppressor | NM_002256 | 213-512 |
| KiSS-1 metastasis-suppressor | NM_002256 | 212-511 |
| H2B histone family, member Q | NM_003528 | 1426-1725 |
| developmentally regulated GTP-binding protein 2 | NM_001388 | 845-1144 |
| TNF receptor-associated factor 6 | NM_004620 | 1635-1934 |
| insulin-like growth factor 1 (somatomedin C) | X57025 | 418-717 |
| coagulation factor II (thrombin) receptor | NM_001992 | 2617-2916 |
| ATP-binding cassette, sub-family B (MDR/TAP), member 1 | NM_000927 | 4183-4482 |
| diaphorase(NADH/NADPH)(cytochrome b-5 reductase) | NM_000903 | 2142-2441 |
| proprotein convertase subtilisin/kexin type 7 | NM_004716 | 2972-3271 |
| seven in absentia (Drosophila) homolog 1 | NM_003031 | 186-485 |
| hepatoma-derived growth factor (high-mobility group protein 1-like) | NM_004494 | 1962-2261 |
| ATP synthase, H+ transporting, mitochondrial F0 complex, subunit b, isoform 1 | NM_001688 | 780-1079 |
| CASP8 and FADD-like apoptosis regulator | Y14039 | 668-967 |
| lumican | NM_002345 | 1239-1538 |
| E74-like factor2(ets domain transcription factor) | NM_006874 | 2045-2344 |
| cytochrome c-1 | NM_001916 | 492-791 |

**Table 41**

| Name of Gene | Accession Number | Selected Gene Region |
|---|---|---|
| plasminogen activator, tissue | NM_000930 | 2153-2452 |
| histone deacetylase 1 | NM_004964 | 1646-1945 |
| DEAD/H (Asp-Glu-Ala-Asp/His) box polypeptide 8 (RNA helicase) | NM_004941 | 3465-3764 |
| signaling lymphocytic activation molecule | NM_003037 | 678-977 |
| phorbol-12-myristate-13-acetate-induced protein1 | NM_021127 | 815-1114 |
| serine protease inhibitor, Kunitz type, 2 | NM_021102 | 776-1075 |
| BCL2-related protein A1 | NM_004049 | 192-491 |
| pleckstrin homology,Sec7 and coiled/coil domains 1(cytohesin 1) | NM_004762 | 2708-3007 |
| ancient ubiquitous protein 1 | NM_012103 | 1348-1647 |
| Rho guanine nucleotide exchange factor (GEF) 1 | NM_004706 | 2387-2686 |
| lamin B2 | M94362 | 3622-3921 |
| v-myb avian myeloblastosis viral oncogene homolog-like 2 | NM_002466 | 1851-2150 |
| lymphoid-restricted membrane protein | NM_006152 | 367-666 |
| myxovirus (influenza) resistance 1, homolog of murine (interferon-inducible protein p78) | NM_002462 | 2274-2573 |
| vitamin D (1,25- dihydroxyvitamin D3) receptor | NM_000376 | 2860-3159 |
| interferon-induced protein with tetratricopeptide repeats 1 | NM_001548 | 1200-1499 |
| guanine nucleotide binding protein (G protein), alpha inhibiting activity polypeptide 3 | NM_006496 | 1798-2097 |

**Table 42**

| Name of Gene | Accession Number | Selected Gene Region |
|---|---|---|
| NIMA (never in mitosis gene a)-related kinase 3 | Z29067 | 1363-1662 |
| UV radiation resistance associated gene | NM_003369 | 3145-3444 |
| zinc finger protein 173 | NM_003449 | 3163-3462 |
| potassium intermediate/small conductance calcium-activated channel,subfamily N, member 3 | NM_002249 | 2027-2326 |
| zinc finger protein homologous to Zfp161 in mouse | D89859 | 1381-1680 |
| guanylate kinase 1 | NM_000858 | 91-390 |
| cell division cycle 2-like 5 (cholinesterase-related cell division controller) | AJ297709 | 4791-5090 |
| profilin 1 | NM_005022 | 145-444 |
| tumor protein p53-binding protein, 1 | NM_005657 | 5803-6102 |
| 3-hydroxymethyl-3-methylglutaryl-Coenzyme A lyase (hydroxymethylglutaricaciduria) | NM_000191 | 80-379 |
| 3-hydroxymethyl-3-methylglutaryl-Coenzyme A lyase (hydroxymethylglutaricaciduria) | NM_000191 | 79-378 |
| putative DNA/chromatin binding motif | AJ243706 | 6087-6386 |
| collagen, type I, alpha 2 | NM_000089 | 4389-4688 |
| hexokinase 1 | NM_000188 | 3191-3490 |
| G-rich RNA sequence binding factor 1 | NM_002092 | 1972-2271 |
| vimentin | NM_003380 | 537-836 |
| serum/glucocorticoid regulated kinase | NM_005627 | 1539-1838 |
| early growth response 1 | NM_001964 | 2658-2957 |
| glutathione S-transferase M4 | NM_000850 | 563-862 |

**Table 43**

| Name of Gene | Accession Number | Selected Gene Region |
|---|---|---|
| guanylate binding protein 1, interferon-inducible, 67kD | NM_002053 | 1602-1901 |
| transforming growth factor, beta receptor II (70-80kD) | D50683 | 4613-4912 |
| Rho GDP dissociation inhibitor (GDI) beta | NM_001175 | 501-800 |
| DNA fragmentation factor, 45kD,alpha polypeptide | NM_004401 | 990-1289 |
| vitiligo-associated protein VIT-1 | NM_018693 | 2623-2922 |
| cyclin D1 (PRAD1: parathyroid adenomatosis 1) | X59798 | 3750-4049 |
| cyclin D1 (PRAD1: parathyroid adenomatosis 1) | X59798 | 3751-4050 |
| retinoblastoma-binding protein 2 | NM_005056 | 2315-2614 |
| Human BRCA2 region, mRNA sequence CG018 | U57962 | 2152-1853 |
| low density lipoprotein-related protein-associated protein 1 (alpha-2-macroglobulin receptor-associated protein 1) | NM_002337 | 1193-1492 |
| low density lipoprotein-related protein-associated protein 1 (alpha-2-macroglobulin receptor-associated protein 1) | NM_002337 | 1192-1491 |
| thyroid autoantigen 70kD (Ku antigen) | NM_001469 | 887-1186 |
| cyclin-dependent kinase 4 | NM_000075 | 851-1150 |
| G1 to S phase transition 1 | NM_002094 | 1693-1992 |
| serine (or cysteine) proteinase inhibitor, clade B (ovalbumin), member 1 | M93056 | 1161-862 |

**Table 44**

| Name of Gene | Accession Number | Selected Gene Region |
|---|---|---|
| tissue inhibitor of metalloproteinase 2 | NM_003255 | 578-877 |
| proliferation-associated 2G4, 38kD | NM_006191 | 1249-1548 |
| developmentally regulated GTP-binding protein 1 | NM_004147 | 667-966 |
| nucleolar phosphoprotein p130 | D21262 | 3156-3455 |
| mesothelin | NM_013404 | 1350-1649 |
| minichromosome maintenance deficient (S. cerevisiae) 4 | X74794 | 2597-2896 |
| signal transducing adaptor molecule (SH3 domain and ITAM motif) 1 | AK024383 | 3726-3427 |
| ubiquitin-like 1 (sentrin) | NM_003352 | 612-911 |
| myeloid cell leukemia sequence 1 (BCL2-related) | AF198614 | 834-1133 |
| keratin 7 | NM_005556 | 1167-1466 |
| keratin 7 | NM_005556 | 1166-1465 |
| non-metastatic cells 1, protein (NM23A) expressed in (NME1), mRNA | NM_000269 | 90-389 |
| major histocompatibility complex, class I, A | NM_002116 | 970-1269 |
| CDC-like kinase 3 | NM_003992 | 1276-1575 |
| cyclin-dependent kinase inhibitor 1B (p27, Kip1) | AY004255 | 1714-2013 |
| keratin 18 | NM_000224 | 705-1004 |
| immunoglobulin heavy constant gamma 3(G3m marker) | Y14737 | 864-1163 |
| PTK2 protein tyrosine kinase 2 | NM_005607 | 1245-1544 |
| interferon, gamma-inducible protein 30 | NM_006332 | 623-922 |

**Table 45**

| Name of Gene | Accession Number | Selected Gene Region |
|---|---|---|
| ribonucleotide reductase M1 polypeptide | NM_001033 | 2333-2632 |
| methylenetetrahydrofolate dehydrogenase (NADP+ dependent), methenyltetrahydrofolate cyclohydrolase,formyltetrahydrofolate synthetase | NM_005956 | 1818-2117 |
| matrix metalloproteinase 19 | NM_002429 | 2455-2754 |
| CDC10 (cell division cycle 10, S. cerevisiae, homolog) | NM_001788 | 1254-1553 |
| transforming growth factor, beta-induced, 68kD | NM_000358 | 2362-2661 |
| Human mRNA for SB classII histocompatibility antigen alpha-chain | X03100 | 781-1080 |
| guanine nucleotide binding protein (G protein), alpha inhibiting activity polypeptide 2 | NM_002070 | 391-690 |
| transforming growth factor beta-stimulated protein TSC-22 | AK027071 | 3370-3071 |
| tumor susceptibility gene 101 | NM_006292 | 664-963 |
| laminin receptor 1 (67kD, ribosomal protein SA) | NM_002295 | 23-322 |
| pM5 protein | NM_014287 | 3671-3970 |
| ras homolog gene family, member A | NM_001664 | 823-1122 |
| polypyrimidine tract binding protein (heterogeneous nuclear ribonucleoprotein I) | NM_002819 | 2560-2859 |
| lactate dehydrogenase A | NM_005566 | 1077-1376 |
| heat shock protein 75 | NM_016292 | 1290-1589 |

**Table 46**

| Name of Gene | Accession Number | Selected Gene Region |
|---|---|---|
| heat shock protein 75 | NM_016292 | 1289-1588 |
| collagen, type VII, alpha 1 (epidermolysis bullosa, dystrophic, dominant and recessive) | NM_000094 | 8962-9261 |
| adenine phosphoribosyltransferase | NM_000485 | 542-841 |
| keratin 8 | NM_002273 | 448-747 |
| anti-oxidant protein 2 (non-selenium glutathione peroxidase, acidic calcium-independent phospholipase A2) | NM_004905 | 1354-1653 |
| connective tissue growth factor | NM_001901 | 1966-2265 |
| insulin-like growth factor binding protein 3 | M35878 | 3082-3381 |
| phosphoglycerate kinase 1 | NM_000291 | 903-1202 |
| ubiquitin-conjugating enzyme E2A (RAD6 homolog) | NM_003336 | 1393-1692 |
| superoxide dismutase 1, soluble (amyotrophic lateral sclerosis 1 (adult)) | NM_000454 | 79-378 |
| cyclin A2 | NM_001237 | 1025-1324 |
| high-mobility group (nonhistone chromosomal) protein 1 | NM_002128 | 562-861 |
| neurotrophic tyrosine kinase, receptor, type 3 | NM_002530 | 859-1158 |
| EphB6 | NM_004445 | 3658-3957 |
| tyrosine 3-monooxygenase/tryptophan 5-monooxygenase activation protein, zeta polypeptide | NM_003406 | 232-531 |
| alkylation repair; alkB homolog | NM_006020 | 1556-1855 |

**Table 47**

| Name of Gene | Accession Number | Selected Gene Region |
|---|---|---|
| keratin 4 | X07695 | 1335-1634 |
| baculoviral IAP repeat-containing 1 | NM_004536 | 4270-4569 |
| nitric oxide synthase 2A(inducible,hepatocytes) | NM_000625 | 3556-3855 |
| nitric oxide synthase 2A(inducible,hepatocytes) | NM_000625 | 3555-3854 |
| p21/Cdc42/Rac1-activated kinase 1 (yeast Ste20-related) | NM_002576 | 1890-2189 |
| elastase 1, pancreatic | NM_001971 | 286-585 |
| elastase 3, pancreatic (protease E) | NM_005747 | 163-462 |
| interferon induced transmembrane protein 2(1-8D) | NM_006435 | 606-905 |
| interferon induced transmembrane protein 2(1-8D) | NM_006435 | 605-904 |
| phospholipase A2, group IB (pancreas) | NM_000928 | 213-512 |
| cadherin 18, type 2 | NM_004934 | 2565-2864 |
| Tubulin, alpha, brain-specific | NM_006009 | 917-1216 |
| phospholipase A2, group VI (cytosolic, calcium-independent) | NM_003560 | 2954-3253 |
| collagen, type VIII, alpha 1 | NM_001850 | 1765-2064 |
| matrix metalloproteinase 2 (gelatinase A, 72kD gelatinase, 72kD type IV collagenase) | NM_004530 | 2496-2795 |
| matrix metalloproteinase 2 (gelatinase A, 72kD gelatinase, 72kD type IV collagenase) | NM_004530 | 2495-2794 |
| microtubule-associated protein, RP/EB family, member 1 | NM_012325 | 1469-1768 |
| interleukin 1, alpha | M28983 | 1211-1510 |

**Table 48**

| Name of Gene | Accession Number | Selected Gene Region |
|---|---|---|
| eukaryotic translation initiation factor 3, subunit 2 (beta, 36kD) | NM_003757 | 585-884 |
| galactosidase, beta 1 | NM_000404 | 2020-2319 |
| B cell RAG associated protein | NM_014863 | 3996-4295 |
| B cell RAG associated protein | NM_014863 | 3995-4294 |
| transforming growth factor, alpha | NM_003236 | 3569-3868 |
| serine protease inhibitor, Kunitz type 1 | NM_003710 | 1800-2099 |
| CD86 antigen(CD28 antigen ligand 2,B7-2 antigen) | NM_006889 | 1069-1368 |
| nuclear factor of kappa light polypeptide gene enhancer in B-cells inhibitor, epsilon | NM_004556 | 1365-1664 |
| interferon, gamma | NM_000619 | 833-1132 |
| O-6-methylguanine-DNA methyltransferase | NM_002412 | 266-565 |
| tyrosine 3-monooxygenase/tryptophan 5-monooxygenase activation protein, zeta polypeptide | NM_003406 | 1425-1724 |
| Homo sapiens cDNA:FLJ20886 fis,clone ADKA03257 | AK024539 | 25-324 |
| Homo sapiens cDNA:FLJ20886 fis,clone ADKA03257 | AK024539 | 24-323 |
| BCL2/adenovirus E1B 19kD-interacting protein 1 | NM_013979 | 542-841 |
| DNAJ domain-containing | NM_013238 | 521-820 |
| RAD51 (S. cerevisiae) homolog C | NM_002876 | 1-300 |
| laminin, alpha 4 | NM_002290 | 4992-5291 |
| ESTs, Moderately similar to endothelial nitric oxide synthase [H.sapiens] | BF666712 | 107-406 |

**Table 49**

| Name of Gene | Accession Number | Selected Gene Region |
|---|---|---|
| ESTs, Moderately similar to endothelial nitric oxide synthase [H.sapiens] | BF666712 | 106-405 |
| vascular endothelial growth factor | AF022375 | 2067-2366 |
| growth differentiation factor 10 | NM_004962 | 1509-1808 |
| MAP/microtubule affinity-regulating kinase 3 | NM_002376 | 1390-1689 |
| MAP/microtubule affinity-regulating kinase 3 | NM_002376 | 1389-1688 |
| retinoic acid receptor, beta | NM_000965 | 1997-2296 |
| cell division cycle 25B | NM_021874 | 1422-1721 |
| erythropoietin receptor | NM_000121 | 617-916 |
| erythropoietin receptor | NM_000121 | 616-915 |
| immunoglobulin lambda locus | X57809 | 1-300 |
| immunoglobulin lambda locus | X57809 | 2-301 |
| fms-related tyrosine kinase 1 (vascular endothelial growth factor/vascular permeability factor receptor) | NM_002019 | 4007-4306 |
| macrophage stimulating 1 (hepatocyte growth factor-like) | AL137798 | 1237-1536 |
| macrophage stimulating 1 (hepatocyte growth factor-like) | AL137798 | 1236-1535 |
| interleukin 1 receptor antagonist | U65590 | 1503-1802 |
| KH-type splicing regulatory protein(FUSE binding protein 2) | NM_003685 | 1822-2121 |

**Table 50**

| Name of Gene | Accession Number | Selected Gene Region |
|---|---|---|
| KH-type splicing regulatory protein (FUSE binding protein 2) | NM_003685 | 1821-2120 |
| junction plakoglobin | NM_002230 | 2157-2456 |
| junction plakoglobin | NM_002230 | 2156-2455 |
| frizzled-related protein | NM_001463 | 627-926 |
| frizzled-related protein | NM_001463 | 626-925 |
| intercellular adhesion molecule 1 (CD54), human rhinovirus receptor | NM_000201 | 2421-2720 |
| intercellular adhesion molecule 1 (CD54), human rhinovirus receptor | NM_000201 | 2420-2719 |
| TGFB inducible early growth response | NM_005655 | 918-1217 |
| jagged 2 | NM_002226 | 1670-1969 |
| jagged 2 | NM_002226 | 1669-1968 |
| cadherin, EGF LAG seven-pass G-type receptor 2, flamingo (Drosophila) homolog | AF234887 | 9344-9643 |
| cadherin, EGF LAG seven-pass G-type receptor 2, flamingo (Drosophila) homolog | AF234887 | 9345-9644 |
| signal transducer and activator of transcription 4 | NM_003151 | 1212-1511 |
| matrix metalloproteinase 12(macrophage elastase) | NM_002426 | 604-903 |
| glycogen synthase kinase 3 beta | NM_002093 | 98-397 |
| glycogen synthase kinase 3 beta | NM_002093 | 99-398 |
| eukaryotic translation initiation factor 2, subunit 2 (beta, 38kD) | NM_003908 | 990-1289 |

**Table 51**

| Name of Gene | Accession Number | Selected Gene Region |
|---|---|---|
| disabled (Drosophila) homolog 2 (mitogen-responsive phosphoprotein) | NM_001343 | 988-1287 |
| disabled (Drosophila) homolog 2 (mitogen-responsive phosphoprotein) | NM_001343 | 989-1288 |
| BTG family, member 2 | NM_006763 | 3-302 |
| nuclear domain 10 protein | NM_005831 | 541-840 |
| FYN oncogene related to SRC, FGR, YES | Z97989 | 331-630 |
| phosphatidylinositol glycan, class F | NM_002643 | 509-808 |
| phosphatidylinositol glycan, class F | NM_002643 | 508-807 |
| RAB36, member RAS oncogene family | NM_004914 | 3377-3676 |
| ESTs | N38956 | 378-79 |
| protein phosphatase 1D magnesium-dependent, delta isoform | NM_003620 | 1656-1955 |
| dihydrofolate reductase | NM_000791 | 1537-1836 |
| ubiquitin-activating enzyme E1-like | NM_003335 | 2533-2832 |
| fibronectin 1 | X02761 | 141-440 |
| hepatocyte growth factor-regulated tyrosine kinase substrate | NM_004712 | 1713-2012 |
| transcription factor Dp-2 (E2F dimerization partner 2) | NM_006286 | 1-300 |
| transcription factor Dp-2 (E2F dimerization partner 2) | NM_006286 | 2-301 |
| mutS (E. coli) homolog 6 | NM_000179 | 2750-3049 |
| DNA (cytosine-5-)-methyltransferase 1 | NM_001379 | 4606-4905 |

**Table 52**

| Name of Gene | Accession Number | Selected Gene Region |
|---|---|---|
| DNA (cytosine-5-)-methyltransferase 1 | NM_001379 | 4605-4904 |
| MAD(mothers against decapentaplegic,Drosophila) homolog 2 | NM_005901 | 1596-1895 |
| fms-related tyrosine kinase 1 (vascular endothelial growth factor/vascular permeability factor receptor) | NM_002019 | 7381-7680 |
| CD22 antigen | NM_001771 | 2273-2572 |
| CD22 antigen | NM_001771 | 2272-2571 |
| folate hydrolase (prostate-specific membrane antigen) 1 | NM_004476 | 1694-1993 |
| jun D proto-oncogene | NM_005354 | 1271-1570 |
| Human glucocorticoid receptor alpha mRNA, variant 3' UTR | U25029 | 1282-1581 |
| laminin, alpha 4 | NM_002290 | 5481-5780 |
| nuclear factor of kappa light polypeptide gene enhancer in B-cells inhibitor, alpha | NM_020529 | 1204-1503 |
| ClpP (caseinolytic protease, ATP-dependent, proteolytic subunit, E. coli) homolog | NM_006012 | 513-812 |
| frizzled-related protein | NM_001463 | 627-926 |
| frizzled-related protein | NM_001463 | 626-925 |
| linker for activation of T cells | NM_014387 | 138-437 |
| guanine nucleotide binding protein (G protein), alpha stimulating activity polypeptide 1 | NM_000516 | 26-325 |
| guanine nucleotide binding protein (G protein), alpha stimulating activity polypeptide 1 | NM_000516 | 25-324 |

**Table 53**

| Name of Gene | Accession Number | Selected Gene Region |
|---|---|---|
| fibroblast growth factor 1 (acidic) | X59065 | 1599-1898 |
| retinoblastoma-binding protein 6 | NM_006910 | 1754-2053 |
| high-mobility group (nonhistone chromosomal) protein 2 | NM_002129 | 474-773 |
| high-mobility group (nonhistone chromosomal) protein 2 | NM_002129 | 473-772 |
| death-associated protein kinase 1 | NM_004938 | 5463-5762 |
| cysteine-rich, angiogenic inducer, 61 | NM_001554 | 1211-1510 |
| keratin 1 (epidermolytic hyperkeratosis) | NM_006121 | 1871-2170 |
| fms-related tyrosine kinase 1 (vascular endothelial growth factor/vascular permeability factor receptor) | NM_002019 | 6664-6963 |
| heterogeneous nuclear ribonucleoprotein K | NM_002140 | 1-300 |
| triple functional domain (PTPRF interacting) | NM_007118 | 7588-7887 |
| adenomatosis polyposis coli | NM_000038 | 8168-8467 |
| BRCA1 associated RING domain 1 | NM_000465 | 1669-1968 |
| BRCA1 associated RING domain 1 | NM_000465 | 1668-1967 |
| granulysin | NM_012483 | 484-783 |
| dishevelled 3 (homologous to Drosophila dsh) | NM_004423 | 2100-2399 |
| splicing factor, arginine/serine-rich 8 (suppressor-of-white-apricot,Drosophila homolog) | NM_004592 | 2785-3084 |

**Table 54**

| Name of Gene | Accession Number | Selected Gene Region |
|---|---|---|
| polymerase (DNA directed), alpha | NM_016937 | 4915-5214 |
| tec protein tyrosine kinase | NM_003215 | 1881-2180 |
| H2B histone family, member Q | NM_003528 | 1191-1490 |
| seven in absentia (Drosophila) homolog 2 | U76248 | 1564-1863 |
| coronin, actin-binding protein, 1A | NM_007074 | 179-478 |
| uridine phosphorylase | NM_003364 | 987-1286 |
| adenosine deaminase | NM_000022 | 1180-1479 |
| growth arrest and DNA-damage-inducible, alpha | L24498 | 2224-2523 |
| receptor (TNFRSF)-interacting serine-threonine kinase 1 | NM_003804 | 729-1028 |
| receptor (TNFRSF)-interacting serine-threonine kinase 1 | NM_003804 | 728-1027 |
| transforming growth factor, beta 2 | NM_003238 | 92-391 |
| transforming growth factor, beta 2 | NM_003238 | 91-390 |
| zinc finger protein, subfamily 1A, 1 (Ikaros) | NM_006060 | 2309-2608 |
| translocated promoter region (to activated MET oncogene) | NM_003292 | 6604-6903 |
| serine/threonine kinase 25(Ste20, yeast homolog) | NM_006374 | 1469-1768 |
| solute carrier family 2 (facilitated glucose transporter), member 3 | NM_006931 | 1598-1897 |
| solute carrier family 2 (facilitated glucose transporter), member 3 | NM_006931 | 1599-1898 |
| ESTs, Highly similar to neuronal apoptosis inhibitory protein [H.sapiens] | AI821682 | 381-82 |

**Table 55**

| Name of Gene | Accession Number | Selected Gene Region |
|---|---|---|
| basigin | NM_001728 | 708-1007 |
| basigin | NM_001728 | 707-1006 |
| cyclin-dependent kinase 5, regulatory subunit 1 (p35) | NM_003885 | 322-621 |
| cyclin-dependent kinase 5, regulatory subunit 1 (p35) | NM_003885 | 323-622 |
| X-ray repair complementing defective repair in Chinese hamster cells 1 | NM_006297 | 1720-2019 |
| tyrosinase-related protein 1 | NM_000550 | 1296-1595 |
| Pseudoautosomal GTP-binding protein-like | NM_012227 | 1557-1856 |
| Pseudoautosomal GTP-binding protein-like | NM_012227 | 1556-1855 |
| antigen identified by monoclonal antibody Ki-67 | X65550 | 12195-12494 |
| antigen identified by monoclonal antibody Ki-67 | X65550 | 12194-12493 |
| neurofibromin 2 (bilateral acoustic neuroma) | NM_000268 | 1899-2198 |
| neurofibromin 2 (bilateral acoustic neuroma) | NM_000268 | 1898-2197 |
| nucleotide binding protein 2 (E.coli MinD like) | NM_012225 | 485-784 |
| nucleotide binding protein 2 (E.coli MinD like) | NM_012225 | 484-783 |
| solute carrier family 25 (mitochondrial carrier; adenine nucleotide translocator), member | NM_001152 | 839-1138 |
| laminin, beta 1 | NM_002291 | 4820-5119 |
| laminin, beta 1 | NM_002291 | 4821-5120 |
| seven in absentia (Drosophila) homolog 1 | NM_003031 | 117-416 |

**Table 56**

| Name of Gene | Accession Number | Selected Gene Region |
|---|---|---|
| high-mobility group (nonhistone chromosomal) protein isoforms I and Y | NM_002131 | 1196-1495 |
| BCL2/adenovirus E1B 19kD-interacting protein 3 | NM_004052 | 313-612 |
| D123 gene product | NM_006023 | 1015-1314 |
| prostate differentiation factor | NM_004864 | 286-585 |
| interferon regulatory factor 3 | NM_001571 | 543-842 |
| interferon regulatory factor 3 | NM_001571 | 544-843 |
| uroporphyrinogen III synthase (congenital erythropoietic porphyria) | NM_000375 | 730-1029 |
| uroporphyrinogen III synthase (congenital erythropoietic porphyria) | NM_000375 | 731-1030 |
| minichromosome maintenance deficient (S. cerevisiae) 2 (mitotin) | NM_004526 | 2969-3268 |
| collagen, type VI, alpha 2 | AL096746 | 1345-1644 |
| restin (Reed-Steinberg cell-expressed intermediate filament-associated protein) | NM_002956 | 4668-4967 |
| restin (Reed-Steinberg cell-expressed intermediate filament-associated protein) | NM_002956 | 4667-4966 |
| v-fos FBJ murine osteosarcoma viral oncogene homolog | NM_005252 | 60-359 |
| v-fos FBJ murine osteosarcoma viral oncogene homolog | NM_005252 | 59-358 |
| tyrosine 3-monooxygenase/tryptophan 5-monooxygenase activation protein, zeta polypeptide | NM_003406 | 232-531 |

**Table 57**

| Name of Gene | Accession Number | Selected Gene Region |
|---|---|---|
| glutathione synthetase | NM_000178 | 1483-1782 |
| retinoid X receptor, alpha | NM_002957 | 4358-4657 |
| tumor necrosis factor, alpha-induced protein 1 (endothelial) | NM_021137 | 1237-1536 |
| tumor necrosis factor, alpha-induced protein 1 (endothelial) | NM_021137 | 1236-1535 |
| GS3955 protein | NM_021643 | 3692-3991 |
| MAD2 (mitotic arrest deficient, yeast, homolog)-like 1 | NM_002358 | 125-424 |
| A kinase (PRKA) anchor protein 1 | NM_003488 | 2434-2733 |
| vaccinia related kinase 2 | NM_006296 | 1010-1309 |
| neutrophil cytosolic factor 1 (47kD, chronic granulomatous disease, autosomal 1) | NM_000265 | 322-621 |
| RAD23 (S. cerevisiae) homolog B | NM_002874 | 2302-2601 |
| postmeiotic segregation increased (S. cerevisiae) 1 | NM_000534 | 2327-2626 |
| vinculin | NM_014000 | 4722-5021 |
| karyopherin alpha 3 (importin alpha 4) | NM_002267 | 1802-2101 |
| thymidylate synthetase | NM_001071 | 1088-1387 |
| thymidylate synthetase | NM_001071 | 1087-1386 |
| chitinase 3-like 2 | NM_004000 | 516-815 |
| protein kinase, cGMP-dependent, type I | NM_006258 | 2516-2815 |

**Table 58**

| Name of Gene | Accession Number | Selected Gene Region |
|---|---|---|
| nuclear factor of activated T-cells,cytoplasmic, calcineurin-dependent 1 | U80917 | 4122-4421 |
| heat shock 70kD protein 4 | AB023420 | 2381-2680 |
| proline-rich protein with nuclear targeting signal | NM_006813 | 885-1184 |
| nuclear factor, interleukin 3 regulated | NM_005384 | 810-1109 |
| quinone oxidoreductase homolog | NM_004881 | 1129-1428 |
| actin, alpha 2, smooth muscle, aorta | NM_001613 | 1-300 |
| actin, alpha 2, smooth muscle, aorta | NM_001613 | 2-301 |
| Fc fragment of IgG, low affinity IIa, receptor for (CD32) | NM_021642 | 1707-2006 |
| sodium bicarbonate transporter 4 | NM_021196 | 921-1220 |
| sodium bicarbonate transporter 4 | NM_021196 | 922-1221 |
| ras-related C3 botulinum toxin substrate 1 (rho family, small GTP binding protein Rac1) | NM_018890 | 62-361 |
| ras-related C3 botulinum toxin substrate 1 (rho family, small GTP binding protein Rac1) | NM_018890 | 61-360 |
| dual-specificity tyrosine-(Y)-phosphorylation regulated kinase 1A | D86550 | 5323-5622 |
| catenin (cadherin-associated protein), alpha 1 (102kD) | NM_001903 | 2813-3112 |
| catenin (cadherin-associated protein), alpha 1 (102kD) | NM_001903 | 2812-3111 |
| v-abl Abelson murine leukemia viral oncogene homolog 1 | NM_005157 | 4953-5252 |

**Table 59**

| Name of Gene | Accession Number | Selected Gene Region |
|---|---|---|
| v-abl Abelson murine leukemia viral oncogene homolog 1 | NM_005157 | 4952-5251 |
| tousled-like kinase 2 | NM_006852 | 2205-2504 |
| protein tyrosine phosphatase, receptor type, F | NM_002840 | 7099-7398 |
| protein tyrosine phosphatase, receptor type, F | NM_002840 | 7098-7397 |
| osteoblast specific factor 2 (fasciclin I-like) | NM_006475 | 1889-2188 |
| preferentially expressed antigen in melanoma | NM_006115 | 1743-2042 |
| phosphoribosylglycinamide formyltransferase, phosphoribosylglycinamide synthetase, phosphoribosylaminoimidazole synthetase | NM_000819 | 2539-2838 |
| early development regulator 2 (homolog of polyhomeotic 2) | NM_004427 | 1897-2196 |
| hypothetical protein FLJ10262 | AB037730 | 4530-4829 |
| bone morphogenetic protein 5 | NM_021073 | 1452-1751 |
| bone morphogenetic protein8(osteogenic protein2) | NM_001720 | 1114-1413 |
| bone morphogenetic protein8(osteogenic protein2) | NM_001720 | 1113-1412 |
| cyclin-dependent kinase inhibitor 2A (melanoma, p16, inhibits CDK4) | NM_000077 | 1-300 |
| cyclin-dependent kinase inhibitor 2A (melanoma, p16, inhibits CDK4) | NM_000077 | 2-301 |
| POU domain, class 2, transcription factor 2 | NM_002698 | 1281-1580 |
| amphiregulin (schwannoma-derived growth factor) | NM_001657 | 80-379 |

**Table 60**

| Name of Gene | Accession Number | Selected Gene Region |
|---|---|---|
| amphiregulin (schwannoma-derived growth factor) | NM_001657 | 79-378 |
| cytochrome c | BF214508 | 1-300 |
| proteasome (prosome, macropain) subunit, beta type, 9 (large multifunctional protease 2) | NM_002800 | 376-675 |
| ephrin-A1 | NM_004428 | 1-300 |
| RAD51(S.cerevisiae)homolog(E coli RecA homolog) | NM_002875 | 499-798 |
| small inducible cytokine subfamily B(Cys-X-Cys), member 10 | NM_001565 | 431-730 |
| CDC16 (cell division cycle 16, S. cerevisiae, homolog) | NM_003903 | 24-323 |
| ras-like protein | NM_012249 | 135-434 |
| ras-like protein | NM_012249 | 134-433 |

In order to prepare a DNA chip on which each of the nucleic acids having a nucleotide sequence of a selected gene region was spotted, an amplified nucleic acid fragment of the gene region was first prepared.

A primer of 20 bases corresponding to an upstream sequence of the specific gene region of each of the genes listed in Tables 4 to 60 and a primer of 20 bases corresponding to a downstream sequence thereof were synthesized using a DNA synthesizer (manufactured by Bio Automation), to give each of primer pairs. PCR was carried out by a conventional method using each of the above primer pairs, to give a desired amplified DNA fragment. Each of the resulting amplified DNA fragments was purified using Qiaquick PCR purification kit 96 (manufactured by QIAGEN) in accordance with the attached protocol.

Each of the above amplified fragments was purified, and thereafter spotted on each of the above slide glass into which an activated carboxyl group was introduced and on TaKaRa Slide Glass (manufactured by Takara Bio Inc.) using the Affymetrix 417 Arrayer (manufactured by Affymetrix).

The slide glass after spotting, into which an activated carboxyl group was introduced was washed with 0.2% SDS, and then washed twice with distilled water. Thereafter, the above slide glass was treated with 0.3 N NaOH for 5 minutes, washed twice with distilled water, and dried by centrifugation (150 × g for 2 minutes). On the other hand, TaKaRa Slide Glass after spotting was subjected to post-treatment in accordance with the instruction manual attached.

The preparation of the labeled cDNA probes, hybridization, washing and analysis were carried out under the same conditions as those described in Example 1.

The analytical results for the genes listed in Tables 4 to 60 in the combinations of the non-labeled substrate/labeled substrate for each of Cy3 and Cy5 are shown in Tables 61 and 62. Table 61 shows the results of a case where the DNA fragment was immobilized on the substrate by electrostatic bonding, i.e., a case where TaKaRa Slide Glass was used. Table 62 shows the results of a case where the DNA fragment was immobilized on the substrate by covalent bonding, i.e., a case where the slide glass into which an activated carboxyl group was introduced was used.

**Table 61**

| | Non-Labeled Substrate/ Labeled Substrate | Cy5 | | |
|---|---|---|---|---|
| | | 5/1 | 3.5/1 | 2/1 |
| | 5/1 | 2.41 | | |
| Cy3 | 3.5/1 | 1.97 | 2.07 | |
| | 2/1 | 1.56 | 1.94 | 2.57 |

**Table 62**

| | Non-Labeled Substrate/ Labeled Substrate | Cy5 | | |
|---|---|---|---|---|
| | | 5/1 | 3.5/1 | 2/1 |
| | 5/1 | 2.01 | | |
| Cy3 | 3.5/1 | 1.86 | 2.13 | |
| | 2/1 | 1.53 | 1.70 | 2.11 |

As shown in Tables 61 and 62, similar results were obtained from both of slide glasses used. In other words, as shown in this example, it was suggested that there are not be influenced by a surface treatment of the substrate or by a binding manner between the nucleic acid to be immobilized and the substrate by using a concentration ratio for the non-labeled substrate/labeled substrate suitable for each of Cy3 and Cy5. In particular, it was found that the analytical results were the most favorable for both of the cases of TaKaRa Slide Glass and the slide glass into which an activated carboxyl group was introduced, when the concentration ratio of a non-labeled substrate/labeled substrate for Cy3 was 2/1 and when the concentration ratio of a non-labeled substrate/labeled substrate for Cy5 was 5/1.

### Example 4

The optimum concentration ratio of the non-labeled substrate/labeled substrate for Cy5 was evaluated by varying a concentration ratio of the non-labeled substrate/labeled substrate for Cy5 within the range of 3/1 to 9/1, with fixing a concentration ratio of the non-labeled substrate/labeled substrate for Cy3 at 2/1. Each of the substrate concentrations is shown in Table 63.

**Table 63**

| Non-Labeled Substrate/Labeled Substrate = 3/1 | |
|---|---|
| dATP | 0.20 mM |
| dGTP | 0.20 mM |
| dCTP | 0.20 mM |
| dTTP | 0.15 mM |
| Cy-dUTP | 0.05 mM |

| Non-Labeled Substrate/Labeled Substrate = 5/1 | |
|---|---|
| dATP | 0.30 mM |
| dGTP | 0.30 mM |
| dCTP | 0.30 mM |
| dTTP | 0.25 mM |
| Cy-dUTP | 0.05 mM |

| Non-Labeled Substrate/Labeled Substrate = 7/1 | |
|---|---|
| dATP | 0.40 mM |
| dGTP | 0.40 mM |
| dCTP | 0.40 mM |
| dTTP | 0.35 mM |
| Cy-dUTP | 0.05 mM |

| Non-Labeled Substrate/Labeled Substrate = 9/1 | |
|---|---|
| dATP | 0.50 mM |
| dGTP | 0.50 mM |
| dCTP | 0.50 mM |
| dTTP | 0.45 mM |
| Cy-dUTP | 0.05 mM |

The preparation of the labeled cDNA probes, hybridization, washing and analytical conditions were carried under the same conditions as those described in Example 1. The studied combinations and the results are shown in Table 64.

**Table 64**

| | Non-Labeled Substrate/ Labeled Substrate | Cy5 | | | |
|---|---|---|---|---|---|
| | | 3/1 | 5/1 | 7/1 | 9/1 |
| ^{Cy3} | 2.0 | 2.01 | 1.60 | 1.82 | 1.72 |

As shown in Table 64, when the concentration ratio of the non-labeled substrate/labeled substrate for Cy3 was fixed at 2/1, it was found that the results of high accuracy were obtained within any of the range of the concentration ratios of the non-labeled substrate/labeled substrate for Cy5 of 5/1 to 9/1, and that especially in a case where the concentration ratio was 5/1, the accuracy became the highest.

### Example 5

The optimum concentration ratio of the non-labeled substrate/labeled substrate for Cy3 was evaluated by varying a concentration ratio of the non-labeled substrate/labeled substrate for Cy3 within the range of 1/1 and 4/1 with fixing a concentration ratio of the non-labeled substrate/labeled substrate for Cy5 at 5/1. Each of the substrate concentrations is shown in Table 65.

**Table 65**

| Non-Labeled Substrate/Labeled Substrate = 1/1 | |
|---|---|
| dATP | 0.10 mM |
| dGTP | 0.10 mM |
| dCTP | 0.10 mM |
| dTTP | 0.05 mM |
| Cy-dUTP | 0.05 mM |

| Non-Labeled Substrate/Labeled Substrate | = 2/1 |
|---|---|
| dATP | 0.15 mM |
| dGTP | 0.15 mM |
| dCTP | 0.15 mM |
| dTTP | 0.10 mM |
| Cy-dUTP | 0.05 mM |

| Non-Labeled Substrate/Labeled Substrate | = 3/1 |
|---|---|
| dATP | 0.20 mM |
| dGTP | 0.20 mM |
| dCTP | 0.20 mM |
| dTTP | 0.15 mM |
| Cy-dUTP | 0.05 mM |

| Non-Labeled Substrate/Labeled Substrate | = 4/1 |
|---|---|
| dATP | 0.25 mM |
| dGTP | 0.25 mM |
| dCTP | 0.25 mM |
| dTTP | 0.20 mM |
| Cy-dUTP | 0.05 mM |

The preparation of the labeled cDNA probes, hybridization, washing and analytical conditions were carried out under the same conditions as those described in Example 1. The studied combinations and the results are shown in Table 66.

**Table 66**

| | Non-Labeled Substrate/ Labeled Substrate | Cy5 | | | |
|---|---|---|---|---|---|
| | | 1/1 | 2/1 | 3/1 | 4/1 |
| Cy5 | 5/1 | 1.73 | 1.48 | 1.53 | 1.54 |

As shown in Table 66, when the concentration ratio of the non-labeled substrate/labeled substrate for Cy5 was fixed at 5/1, the concentration ratios of the non-labeled substrate/labeled substrate for Cy3 were preferably 2/1 or higher, and the results of high accuracy were obtained with any of the concentration ratios of 2/1, 3/1, and 4/1. In particular, it was found that it is preferable to set to have as low concentration ratio of the non-labeled substrate/labeled substrate for Cy3 as possible, in order to increase the ratio of significant spots, and it is particularly preferable to have the non-labeled substrate/labeled substrate of 2/1.

### Example 6

Studies were made on the method of the present invention with varying the kinds of reverse transcriptases. In this example, a reverse transcriptase derived from AMV (Avian myeloblastosis virus) (manufactured by Takara Bio Inc.) was used. Also, the concentration ratio for the non-labeled substrate/labeled substrate was set at 1.86/1 for both Cy3 and Cy5, or set at 2/1 for Cy3 and 5/1 for Cy5, and the analytical results were compared. Each of the substrate concentrations is shown in Table 67. The studied combinations of the concentration ratios of the fluorescent substance and the non-labeled substrate/labeled substrate and the results are shown in Table 68. Here, the preparation of the labeled cDNA probes, hybridization with the DNA microarray, washing, scanning and analysis were carried out under the same conditions as those described in Example 1.

**Table 67**

| Non-Labeled Substrate/Labeled Substrate = 5/1 | |
|---|---|
| dATP | 0.30 mM |
| dGTP | 0.30 mM |
| dCTP | 0.30 mM |
| dTTP | 0.25 mM |
| Cy-dUTP | 0.05 mM |

| Non-Labeled Substrate/Labeled Substrate = 1.86/1 | |
|---|---|
| dATP | 0.1 mM |
| dGTP | 0.1 mM |
| dCTP | 0.1 mM |
| dTTP | 0.065 mM |
| Cy-dUTP | 0.035 mM |

| Non-Labeled Substrate/Labeled Substrate = 2/1 | |
|---|---|
| dATP | 0.15 mM |
| dGTP | 0.15 mM |
| dCTP | 0.15 mM |
| dTTP | 0.10 mM |
| Cy-dUTP | 0.05 mM |

**Table 68**

| Cy3 | Cy5 | Analytical Accuracy |
|---|---|---|
| Non-Labeled Substrate/Labeled Substrate | | |
| 1.86/1 | 1.86/1 | 2.89 |
| 2.0/1 | 5.0/1 | 1.46 |

As shown in Table 68, when the concentration ratios of the non-labeled substrate/labeled substrate for both Cy3 and Cy5 were fixed at the same 1.86/1, a Cy3/Cy5 signal ratio at a 99% convergence in distribution of the significant spots (i.e., analytical accuracy) was shown to have a value of 2.89. When the concentration ratios of the non-labeled substrate/labeled substrate were fixed at 2/1 for Cy3 and 5/1 for Cy5, the Cy3/Cy5 signal ratio (i.e., analytical accuracy) became 1.46. Therefore, it was found that the improvement in the analytical accuracy was seen even when the AMV-derived reverse transcriptase was used.

In other words, it was found that as a result of gene expression analysis using a DNA chip or DNA microarray, the expression of all of the genes approximated 1:1 when the same mRNA of the same amount was labeled with each of the fluorescent markers Cy3 and Cy5 by setting the concentration ratio of the labeled substrate/non-labeled substrate separately for Cy3 and Cy5, even when the reverse transcriptase derived from AMV was used.

### INDUSTRIAL APPLICABILITY

According to the labeling method of the present invention, there can be provided a labeled nucleic acid capable of performing analysis with reflecting the inherent profile of gene expression. Therefore, according to the labeling method of the present invention, there can be accurately grasped the behavior of the gene expressions in gene expression analysis. Also, according to the present invention, there can be provided a kit for fluorescent-labeling a probe capable of performing the gene expression analysis using the dual color hybridization method at high accuracy.

## Claims

1. A method for labeling a nucleic acid, wherein the method is a method for labeling the nucleic acid with at least two kinds of different labeled substances distinguishable from each other, and wherein the method comprises the step of labeling the nucleic acid in a nucleic acid sample containing plural kinds of nucleic acids by use of:
one labeled substrate which is labeled with a labeling substance and
a non-labeled substrate corresponding thereto,
in an amount ratio satisfying the following conditions that a ratio of:
a) a signal intensity of a signal ascribed to a labeled nucleic acid prepared with the nucleic acid in the nucleic acid samples as a template, wherein the labeled nucleic acid is labeled with the labeled substrate, to
b) a signal intensity of a signal ascribed to a labeled nucleic acid prepared with the same nucleic acid as that of the above a) as a template, wherein the labeled nucleic acid is labeled with a labeled substrate different from the labeled substrate of the above a)
in each of the nucleic acids in the nucleic acid sample is substantially the same, irrelevant to the kinds of the nucleic acids to be used as a template.

2. The method according to claim 1, wherein the labeled nucleic acid is prepared by reverse transcription reaction from the nucleic acid used as a template.

3. The method according to claim 1 or 2, wherein the different labeled substrate is Cy3-labeled substrate or Cy5-labeled substrate.

4. The method according to claim 3, wherein the nucleic acid in the nucleic acid sample is labeled in a reaction mixture containing the non-labeled substrate and the Cy3-labeled substrate in a concentration ratio (the non-labeled substrate/the Cy3-labeled substrate) within the range of from 1/1 to 5/1.

5. The method according to claim 3 or 4, wherein the nucleic acid in the nucleic acid sample is labeled in a reaction mixture containing the non-labeled substrate and the Cy5-labeled substrate in a concentration ratio (the non-labeled substrate/the Cy5-labeled substrate) within the range of from 3/1 to 10/1.

6. A labeled nucleic acid prepared by the method of any one of claims 1 to 5.

7. A kit for labeling a nucleic acid comprising an instruction manual describing the procedures of the method of any one of claims 1 to 5.

8. The kit according to claim 7, wherein the instruction manual describes a method for preparing a mixed substrate containing the Cy3-labeled substrate and the non-labeled substrate corresponding thereto in a concentration ratio (the non-labeled substrate/the Cy3-labeled substrate) within the range of from 1/1 to 5/1.

9. The kit according to claim 7 or 8, wherein the instruction manual describes a method for preparing a mixed substrate containing the Cy5-labeled substrate and the non-labeled substrate corresponding thereto in a concentration ratio (the non-labeled substrate/the Cy5-labeled substrate) within the range of from 3/1 to 10/1.

10. A kit for labeling a nucleic acid, comprising:
(1) a reaction vessel containing a reaction mixture in an amount of one-time use or defined times of use, wherein the reaction mixture comprises a Cy3-labeled substrate and a non-labeled substrate corresponding thereto in a concentration ratio (non-labeled substrate/Cy3-labeled substrate) of from 1/1 to 5/1, and comprises a non-labeled nucleotide substrate other than the above non-labeled substrate, and/or
(2) a reaction vessel containing a reaction mixture in an amount for one-time use or defined times of use, wherein the reaction mixture comprises a Cy5-labeled substrate and a non-labeled substrate corresponding thereto in a concentration ratio (non-labeled substrate/Cy5-labeled substrate) within the range of from 3/1 to 10/1, and comprises a non-labeled nucleotide substrate other than the above non-labeled substrate.

11. The kit according to claim 10, wherein the reaction mixture further comprises a reverse transcriptase.
